# EUROPÄISCHE PATENTSCHRIFT

(11) **EP 4 267 066 B1**
(45) Veröffentlichungstag und Bekanntmachung des Hinweises auf die Patenterteilung: **10.09.2025**
(21) Anmeldenummer: 21844649.0
(22) Anmeldetag: 21.12.2021
(51) Int. Cl.: A61F 13/532, A61F 13/534, A61F 13/537, A61F 13/84

(54) **INKONTINENZARTIKEL MIT PH-REGULATIONSMITTEL**
INCONTINENCE ARTICLE WITH PH REGULATOR
ARTICLE POUR INCONTINENCE AVEC RÉGULATEUR DE PH

(30) Priorität: 23.12.2020 DE 102020134790
(43) Veröffentlichungstag der Anmeldung: 01.11.2023
(73) Patentinhaber: Paul Hartmann AG, 89522 Heidenheim (DE)
(72) Erfinder: VECHTER, Olga, 89075 Ulm (DE); BÄHRLE, Christian, 89542 Herbrechtingen (DE); KESSELMEIER, Rüdiger, 89233 Burlafingen (DE); GAUSE, Enno, 89522 Heidenheim (DE)
(74) Vertreter: Paul Hartmann AG Patents & Licensing
(86) Internationale Anmeldenummer: PCT/EP2021/086963
(87) Internationale Veröffentlichungsnummer: WO 2022/136358

(56) Entgegenhaltungen:
- WO-A1-2019/167356
- WO-A1-2021/123085
- DE-T2- 3 686 903
- US-A1- 2011 224 637
- US-B2- 9 757 285

## Beschreibung

Die vorliegende Erfindung betrifft einen Inkontinenzartikel für die Aufnahme von Körperausscheidungen mit einem Mittel zur Regulation des pH-Wertes für die bevorzugte Verwendung durch Erwachsene.

Ein häufiges Problem bei der Verwendung von Inkontinenzartikeln ist das Auftreten von Hautreizungen oder Hautentzündungen, welche durch den Kontakt der Haut mit Körperausscheidungen wie beispielsweise Urin hervorgerufen werden. Oftmals kann sich eine sogenannte Inkontinenz-assoziierte Dermatitis (IAD) entwickeln, eine durch Kontakt mit einem Reizstoff wie Urin hervorgerufene Hautentzündung in der perinealen oder perigenitalen Region. Dabei spielen Veränderungen des pH-Wertes der Haut bei Kontakt mit dem alkalischeren Urin eine wesentliche Rolle. Um einen solchen Kontakt der Haut mit Urin zu verringern weisen Inkontinenzartikel typischerweise superabsorbierende Materialien auf, welche große Mengen an Urin aufnehmen und dauerhaft binden können. Außerdem kann mit einem entsprechenden Aufbau des Inkontinenzartikels, beispielsweise durch die Verwendung von Flüssigkeitsaufnahme- und Verteilerschichten im Inneren des Inkontinenzartikels, Flüssigkeiten schneller in den Inkontinenzartikel aufgenommen und dabei von der Haut des Benutzers weggeleitet werden. Teilweise verfügen Inkontinenzartikel auch über Öffnungen, Rillen oder Vertiefungen, welche zu einer besseren Flüssigkeitsleitung und -verteilung im Inkontinenzartikel beitragen sollen. Weiterhin werden manchmal Substanzen in das absorbierende Material des Saugkörpers eingebracht, um den pH-Wert der aufgenommenen Körperflüssigkeiten zu regulieren.

Jedoch kommt es häufig vor, dass bei einem Miktionsereignis eine große Menge an Urin in kurzer Zeit, also schwallartig, auf den Inkontinenzartikel trifft, so dass der Artikel diese Menge nicht schnell genug ableiten kann. Dabei kann sich der Urin zunächst im Schrittbereich des Inkontinenzartikels sammeln oder sich auf der Oberfläche des Saugkörpers vom Auftreffpunkt ausgehend in die Fläche ausbreiten, bevor er vom Saugkörper vollständig aufgesogen werden kann. Dadurch kommt es zu einem längeren und großflächigen Hautkontakt, so dass leicht Hautreizungen entstehen können. Es besteht also weiterhin Bedarf an verbesserten Inkontinenzartikeln.

Inkontinenzartikel für Erwachsene sind seit langem bekannt und weisen häufig ein zumindest bereichsweise flüssigkeitsdurchlässiges Topsheet, ein im Wesentlichen flüssigkeitsundurchlässiges Backsheet und einen zwischen dem Topsheet und dem Backsheet angeordneten Absorptionskörper auf. Diese können grundsätzlich auf vielfältige Weise ausgestaltet sein, beispielsweise als direkt am Körper anliegende Inkontinenzprodukte oder als saugfähige Inkontinenzunterlagen.

Im Stand der Technik finden sich Ansätze, Inkontinenzartikel mit einem Mittel zur pH-Wert-Kontrolle bereitzustellen. DE3686903T2 lehrt einen absorbierenden Artikel, der einen oder mehrere pH-Regulierungswirkstoffe und ein flüssigkeitsabsorbierendes, hochneutralisiertes Hydrogel-Material aufweist. Hydrogel und pH-Regulierungswirkstoff sollen dabei uneinheitlich verteilt und in getrennten Zonen oder Schichten des Absorptionskörpers angeordnet sein. WO2019167356A1 lehrt einen absorbierenden Artikel mit einem pH-Regulierungswirkstoff in einer dem Körper abgewandten Lage des Absorptionskörpers. Der pH-Regulierungswirkstoff besteht aus einem festen Kern mit sauren Eigenschaften und einer Fett-artigen Beschichtung. Die Beschichtung soll die Freisetzung des pH-Regulierungswirkstoffes verlangsamen. WO2012121932A1 lehrt einen absorbierenden Artikel mit einer Pufferzusammensetzung, die mit absorbierendem Material vorgemischt ist oder die zwischen zwei Saugkörperschichten angeordnet ist. Dabei soll die Pufferzusammensetzung im trockenen Zustand jedenfalls keinen Kontakt zum Topsheet haben.

Die Erfinder haben erkannt, dass eine solche Anordnung den Nachteil hat, dass die Körperflüssigkeit, die beim Eindringen in den absorbierenden Kern in Kontakt mit der Pufferzusammensetzung kommt, diese beim Vordringen in tiefere Saugkörperlagen ausschwemmen kann oder überhaupt erst in tieferen Saugkörperlagen mit der Pufferzusammensetzung in Kontakt kommt. Außerdem kann bei einem Anfall einer großen Menge an Körperflüssigkeit diese häufig nicht schnell genug in den Saugkörper aufgesogen werden, so dass sich ein Teil der Flüssigkeit ohne Kontakt zu den die Pufferzusammensetzung enthaltenden Saugkörperlagen an der Oberfläche des Artikels ausbreiten kann. Die Kontrolle des pH-Wertes an einer hautseitigen Oberfläche des Artikels kann daher insbesondere bei schwallartigem Auftreffen von Körperflüssigkeiten auf den Inkontinenzartikel ungleichmäßig und/oder nur in ungenügendem Maße erfolgen.

Der vorliegenden Erfindung liegt daher die Aufgabe zu Grunde, diese Nachteile zu überwinden.

Diese Aufgabe wird gelöst durch Inkontinenzartikel für die Aufnahme von Körperausscheidungen, umfassend ein zumindest bereichsweise flüssigkeitsdurchlässiges Topsheet, ein im Wesentlichen flüssigkeitsundurchlässiges Backsheet und einen zwischen dem Topsheet und dem Backsheet angeordneten Absorptionskörper wobei der Absorptionskörper eine erste Saugkörperlage aufweist, und wobei ein pH-Regulationsmittel ungleichförmig in die erste Saugkörperlage eingebracht ist, so dass ein mittlerer Bereich des Absorptionskörpers und ein vorderer Bereich des Absorptionskörpers und ein hinterer Bereich des Absorptionskörpers pH-Regulationsmittel enthalten, derart, dass eine flächenbezogene Menge des pH-Regulationsmittels in dem mittleren Bereich des Absorptionskörpers in g/m² größer ist als in dem vorderen und in dem hinteren Bereich des Absorptionskörpers.

Der vordere, mittlere und hintere Bereich des Absorptionskörpers sind vorzugsweise auf einer Längsmittelachse des Absorptionskörpers zu verorten.

Der vordere Bereich ist dabei näher an einer vorderen, also im Benutzungszustand bauchseitig zu liegen kommenden Querkante des Absorptionskörpers zu verorten als der mittlere und der hintere Bereich. Der hintere Bereich ist näher an einer hinteren, also im Benutzungszustand rückenseitig zu liegen kommende Querkante des Absorptionskörpers zu verorten als der mittlere und vordere Bereich. Der mittlere Bereich ist bevorzugt in dem Miktionsbereich zu verorten, also in dem Bereich, in dem im Benutzungszustand mit hoher Wahrscheinlichkeit Körperflüssigkeiten wie Urin auf den Inkontinenzartikel auftreffen.

Durch eine solche ungleichförmige Verteilung des pH-Regulationsmittels wird auf vorteilhafte Weise erreicht, dass das pH-Regulationsmittel vorrangig in einem Bereich des ersten Auftreffens von Körperflüssigkeiten wie beispielsweise Urin (Miktionsbereich) auf den Inkontinenzartikel bereitgestellt ist, sodass auch beim Anfall großer Flüssigkeitsmengen eine effektive pH-Kontrolle ermöglicht wird.

Gleichzeitig wird erreicht, dass auch - relativ zu dem mittleren Bereich - weiter vorn und/oder weiter hinten pH-Regulationsmittel in dem Absorptionskörper vorhanden ist, wodurch eine pH-Kontrolle über einen längeren Zeitraum und in vom Miktionsbereich weiter entfernten Regionen des Absorptionskörpers verbessert wird. Insbesondere bei einem schwallartigen Auftreffen großer Urinmengen kann es nämlich dazu kommen, dass sie Flüssigkeit sich an der Oberfläche des Inkontinenzartikels oder des Absorptionskörpers vom Miktionsbereich ausgehend in die Fläche ausbreitet, bevor sie vom Absorptionskörper vollständig aufgenommen werden kann.

Bevorzugt beträgt die flächenbezogene Menge an pH-Regulationsmittel in dem mittleren Bereich 5 bis 100 g/m², insbesondere 10 bis 50 g/m², weiter insbesondere 15 bis 30 g/m².

Weiter bevorzugt beträgt die flächenbezogene Menge an pH-Regulationsmittel in dem vorderen und/oder hinteren Bereich 0,1-50,0 g/m², insbesondere 0,2-40,0 g/m², weiter insbesondere 0,3-30,0 g/m², weiter insbesondere 0,5-20,0 g/m².

In einem bevorzugten Ausführungsbeispiel ist die flächenbezogene Menge des pH-Regulationsmittels in einem mittleren Längsabschnitt des Absorptionskörpers in g/m² größer als in einem vorderen und/oder einem hinteren Längsabschnitt des Absorptionskörpers.

Der mittlere Längsabschnitt des Absorptionskörpers ist hierbei in einem Schrittbereich des Inkontinenzartikels, mithin den Punkt oder Bereich des Auftreffens von Urin bei der Benutzung (Miktionsbereich) überfangend angeordnet und erstreckt sich über eine Quermittelachse des Absorptionskörpers hinweg. Der vordere Längsabschnitt des Absorptionskörpers schließt sich in Längsrichtung unmittelbar an den mittleren Längsabschnitt in Richtung der vorderen Querkante des Absorptionskörpers an und erstreckt sich bis zu der vorderen Querkante des Absorptionskörpers.

Der hintere Längsabschnitt des Absorptionskörpers schließt sich in Längsrichtung unmittelbar an den mittleren Längsabschnitt in Richtung der hinteren Querkante des Absorptionskörpers an und erstreckt sich bis zu der hinteren Querkante des Absorptionskörpers. Die Grenzen zwischen mittlerem und vorderem Längsabschnitt und zwischen mittlerem und hinterem Längsabschnitt verlaufen jeweils geradlinig in Querrichtung des Inkontinenzartikels und orthogonal zu einer Längsmittelachse des Absorptionskörpers. Der mittlere Längsabschnitt des Absorptionskörpers ist hinsichtlich seiner Längserstreckung symmetrisch zu der Quermittelachse des Absorptionskörpers angeordnet.

Eine Längserstreckung des mittleren Längsabschnitts des Absorptionskörpers beträgt im Rahmen der vorliegenden Erfindung 30-65 % einer Gesamtlängserstreckung des Absorptionskörpers. Das bedeutet, dass in einem beliebigen Längsabschnitt, dessen Längserstreckung 30-65% einer Gesamtlängserstreckung des Absorptionskörpers beträgt und der hinsichtlich seiner Längserstreckung symmetrisch zu der Quermittelachse des Absorptionskörpers angeordnet ist, die flächenbezogene Menge des pH-Regulationsmittels in diesem mittleren Längsabschnitt in g/m² größer ist als in einem jeweiligen vorderen und/oder einem hinteren Längsabschnitt des Absorptionskörpers. Insbesondere beträgt die Längserstreckung des mittleren Längsabschnitts des Absorptionskörpers 30-55 %, weiter insbesondere 35-50%, weiter insbesondere 40-45 %, weiter insbesondere 43 % der Gesamtlängserstreckung des Absorptionskörpers.

Der mittlere, hintere und vordere Längsabschnitt des Absorptionskörpers erstreckt sich in Querrichtung des Absorptionskörpers über eine gesamte Breite des Absorptionskörpers, mithin von einer ersten Längskante bis zu einer zweiten Längskante.

Als Längserstreckung ist zu verstehen eine maximale Längserstreckung eines Elementes oder Längsabschnitts.

Als Quererstreckung ist zu verstehen eine maximale Quererstreckung eines Elementes oder Längsabschnitts.

Der Absorptionskörper kann rechteckig, dreieckig, oval, T-förmig, sanduhrförmig, anatomisch, asymmetrisch oder in einer anderen dem Fachmann als geeignet erscheinenden Form ausgebildet sein.

Das Backsheet kann insbesondere ein atmungsaktives, jedoch im Wesentlichen flüssigkeitsdichtes Folienmaterial umfassen oder dadurch gebildet sein. Das Topsheet ist zumindest bereichsweise vorzugsweise von einem auf Vliesbasis beruhenden zumindest bereichsweise flüssigkeitsdurchlässigen Material gebildet.

Bevorzugt weisen der vordere und/oder der hintere Längsabschnitt des Absorptionskörpers pH-Regulationsmittel auf. Dadurch können auch Teilmengen von Urin rasch pH-reguliert werden, die sich bei einem schwallartigen Auftreffen gegebenenfalls zunächst an der Oberfläche des Inkontinenzartikels ausbreiten und die erst im hinteren oder vorderen Längsabschnitt des Absorptionskörpers aufgenommen werden.

Es ist auch denkbar, dass der vordere und/oder hintere Längsabschnitt des Absorptionskörpers im Wesentlichen frei von pH-Regulationsmittel sind.

In einem bevorzugten Ausführungsbeispiel ist 5,00-30,00 g/m², insbesondere 6,00-25,00 g/m², weiter insbesondere 7,00-20,00 g/m², weiter insbesondere 8,00-15,00 g/m² pH-Regulationsmittel im mittleren Längsabschnitt des Absorptionskörpers angeordnet. Weiter bevorzugt ist im vorderen und/oder hinteren Längsabschnitt des Absorptionskörpers jeweils 0,00-10,00 g/m², insbesondere 0,10-9,00 g/m², weiter insbesondere 0,20-7,00 g/m², weiter insbesondere 0,30-5,00 g/m² pH-Regulationsmittel angeordnet.

In einer bevorzugten Ausführungsform ist das pH-Regulationsmittel in der ersten Saugkörperlage derart eingebracht, dass die Konzentration des pH-Regulationsmittels in Gewichtsprozent in einem mittleren Längsabschnitt des Absorptionskörpers größer ist als in einem vorderen und/oder einem hinteren Längsabschnitt des Absorptionskörpers. Hierdurch kann auf vorteilhafte Weise die Menge (Gesamtmenge und/oder flächenbezogene Menge) an pH-Regulationsmittel in dem mittleren Längsabschnitt unabhängig von einer Erhöhung einer Gesamtmaterialmenge des Absorptionskörpers und damit einer Dicken- oder einer Dichteänderung im mittleren Längsabschnitt erhöht werden.

Hierbei ist zu erwähnen, dass der Absorptionskörper und/oder die erste Saugkörperlage eine gleichförmige Dicke und/oder eine gleichförmige Dichte aufweisen kann. Ebenso ist denkbar, dass der Absorptionskörper und/oder die erste Saugkörperlage ein Dicken- oder Höhenprofil oder ein Dichteprofil aufweist.

Das Einbringen des pH-Regulationsmittels in die erste Saugkörperlage kann auf unterschiedliche Arten erfolgen. Bevorzugt wird das pH-Regulationsmittel auf einer Oberfläche der ersten Saugkörperlage angeordnet, insbesondere auf einer dem Topsheet zugewandten Oberfläche der ersten Saugkörperlage. Denkbar ist jedoch auch, dass das pH-Regulationsmittel im Inneren der ersten Saugkörperlage, dabei entlang der Dickenrichtung der ersten Saugkörperlage gleichförmig oder ungleichförmig angeordnet wird.

Nach einer bevorzugten Ausführungsform ist ein mittlerer Bereich des Absorptionskörpers in dem mittleren Längsabschnitt des Absorptionskörpers zu verorten. Nach einer weiteren vorteilhaften Ausführungsform ist ein vorderer Bereich des Absorptionskörpers in dem vorderen Längsabschnitt des Absorptionskörpers zu verorten. Ein vorderer Bereich des Absorptionskörpers kann zusätzlich oder alternativ aber auch ganz oder abschnittsweise in dem mittleren Längsabschnitt des Absorptionskörpers zu verorten sein. Nach einer weiteren vorteilhaften Ausführungsform ist ein hinterer Bereich des Absorptionskörpers in dem hinteren Längsabschnitt des Absorptionskörpers zu verorten. Ein hinterer Bereich des Absorptionskörpers kann zusätzlich oder alternativ aber auch ganz oder abschnittsweise in dem mittleren Längsabschnitt des Absorptionskörpers zu verorten sein.

Der Absorptionskörper des Inkontinenzartikels ist geeignet und dazu bestimmt, Körperausscheidungen, insbesondere Körperflüssigkeiten, insbesondere Urin aufzunehmen und dauerhaft zu speichern.

Dazu weist die erste Saugkörperlage bevorzugt superabsorbierendes Polymer (SAP) auf.

In einer vorteilhaften Ausführungsform weist die erste Saugkörperlage einen ersten Bereich auf, wobei das SAP in dem ersten Bereich der ersten Saugkörperlage derart eingebracht ist, dass die Konzentration des SAP in Gewichtsprozent in einem mittleren Längsabschnitt des ersten Bereichs der ersten Saugkörperlage gleich groß ist wie in einem vorderen und/oder einem hinteren Längsabschnitt des ersten Bereichs der ersten Saugkörperlage.

Hierdurch ergeben sich Vorteile bei der Herstellung, da beispielsweise das SAP mit dem weiteren Material des ersten Bereichs der ersten Saugkörperlage gleichförmig vorgemischt werden kann und eine schwierigere separate Dosierung und/oder Anordnung des SAP bei hoher Maschinengeschwindigkeit entfallen kann.

In einer bevorzugten Ausführungsform enthält der Absorptionskörper superabsorbierendes Polymermaterial (SAP) zu 5 - 100 Gewichtsprozent, vorzugsweise zu 10 - 95 Gewichtsprozent, weiter vorzugsweise zu 15 - 90 Gewichtsprozent, ganz besonders bevorzugt zu 20 - 80 Gewichtsprozent. Typischerweise kann das SAP-Material zumindest das 15-fache, insbesondere das 20-fache seines Gewichts an 0,9 gewichtsprozentiger Kochsalzlösung absorbieren (gemessen nach NWSP 242.0.R2(15)).

Das SAP kann beispielsweise partikelförmig oder faserförmig oder blatt- oder schaumförmig ausgebildet sein.

Das SAP kann weiterhin ein im Wesentlichen sortenreines Polymermaterial oder eine Mischung von zwei oder mehreren Polymermaterialien umfassen oder daraus bestehen.

Der Absorptionskörper kann weitere Materialien, wie Zellstofffasern ("fluff") oder Kunststofffasern enthalten. Denkbar ist weiterhin, die erste und/oder die nachfolgend näher beschriebene zweite Saugkörperlage des Absorptionskörpers durch Anordnung von ein oder mehreren Schichten verschiedenen Materials, insbesondere aus Vliesmaterial auszubilden.

In einer bevorzugten Ausführung weist die erste Saugkörperlage einen in der Längsrichtung orientierten Kanal auf, wobei zumindest eine Teilmenge des pH-Regulationsmittels in den Kanal eingebracht ist.

Da der Kanal der primären Aufnahme von Körperflüssigkeiten wie Urin und deren anschließenden Weiterleitung in weiter entfernte Regionen des Absorptionskörpers dient, unterstützt das im Kanal eingebrachte pH-Regulationsmittel eine schnelle pH-Kontrolle, so dass der pH-Wert reguliert werden kann, bevor die Körperflüssigkeiten im Absorptionskörper und insbesondere durch SAP gebunden werden.

Bevorzugt ist der Kanal parallel zu einer Längsmittelachse des Absorptionskörpers und/oder der ersten Saugkörperlage und/oder des Inkontinenzartikels angeordnet.

Insbesondere ist der Kanal auf einer Längsmittelachse des Inkontinenzartikels und/oder des Absorptionskörpers und/oder der ersten Saugkörperlage angeordnet.

Weiter insbesondere ist der Kanal symmetrisch zur Längsmittelachse des Inkontinenzartikels und/oder des Absorptionskörpers und/oder der ersten Saugkörperlage ausgebildet.

Der Kanal ist auf vorteilhafte Weise in Querrichtung des Absorptionskörpers und/oder der ersten Saugkörperlage zentriert angeordnet.

Vorteilhafterweise ist der Kanal in Längsrichtung des Inkontinenzartikels im Bereich des Auftreffens von Urin bei der Benutzung angeordnet.

Weiterhin erstreckt sich der Kanal in vorteilhafter Weise über die Quermittelachse des Absorptionskörpers hinweg.

Ein bevorzugter Kanal ist derart angeordnet, dass er von der hinteren Querkante des Absorptionskörpers weiter entfernt ist als von der vorderen Querkante des Absorptionskörpers. Jedoch kann der Kanal auch gleich weit von der vorderen und hinteren Querkante des Absorptionskörpers entfernt oder weiter von der vorderen als von der hinteren Querkante des Absorptionskörpers entfernt angeordnet sein.

In einer bevorzugten Ausführungsform erstreckt sich der Kanal geradlinig in der Längsrichtung des Inkontinenzartikels und/oder des Absorptionskörpers und/oder der ersten Saugkörperlage. Dadurch kann die Flüssigkeit sich im Kanal ungehinderter in der Längsrichtung des Inkontinenzartikels ausbreiten.

Jedoch sind auch wellenförmige, gezackte oder bogenförmige oder jede andere Kanalform denkbar, die der Fachmann für sinnvoll erachtet.

Unterschiedliche Kanalformen wie beispielsweise rechteckig, mandelförmig, knochenförmig, T-förmig, dreieckig, oval, sternförmig oder verzweigt sind im Stand der Technik hinreichend bekannt.

Die Erfinder haben festgestellt, dass für eine rasche Flüssigkeitsleitung in Längsrichtung jedoch gerade solche Kanäle vorteilhaft sind, deren Erstreckung in der Längsrichtung des Inkontinenzartikels größer ist als deren Erstreckung in Querrichtung. Insbesondere rechteckige, mandelförmige oder ovale Kanäle haben sich als vorteilhaft erwiesen.

In einer bevorzugten Ausführungsform umfasst der Kanal eine sich durch die erste Saugkörperlage in Dickenrichtung hindurcherstreckende Ausnehmung oder ist daraus gebildet.

Dabei ist der Kanal bevorzugt im Wesentlichen frei von absorbierenden Materialien. Dazu kann der Kanal durch Weglassen des absorbierenden Materials oder durch Entfernen von absorbierendem Material mittels Schneiden oder Stanzen ausgebildet werden.

Ebenso denkbar ist jedoch, dass der Kanal durch Prägen oder Komprimieren der ersten Saugkörperlage ausgebildet wird.

Nach einer bevorzugten Ausführungsform weist die erste Saugkörperlage einen einzigen Kanal auf.

In einer alternativen Ausführungsform weist die erste Saugkörperlage mehr als einen Kanal, insbesondere 2-5, weiter insbesondere genau 2 Kanäle auf.

In einer bevorzugten Ausführungsform grenzt der erste Bereich an den Kanal an, insbesondere umgibt der erste Bereich den Kanal vollständig und zumindest eine Teilmenge des SAP, insbesondere die gesamte Menge des SAP der ersten Saugkörperlage ist in den ersten Bereich eingebracht. Das ermöglicht, dass der pH-Wert der in den Kanal aufgenommenen Körperflüssigkeit, insbesondere Urin, bereits vor einer dauerhaften Bindung durch SAP reguliert werden kann.

In einer weiteren bevorzugten Ausführungsform weist der Absorptionskörper eine zweite Saugkörperlage auf, wobei die zweite Saugkörperlage unterhalb der ersten Saugkörperlage angeordnet ist, also zwischen erster Saugkörperlage und Backsheet.

Weiter bevorzugt weist die zweite Saugkörperlage weniger als 20 Gewichtsprozent, 15 Gewichtsprozent, 10 Gewichtsprozent, 5 Gewichtsprozent SAP auf, insbesondere ist die zweite Saugkörperlage frei von SAP.

Die zweite Saugkörperlage bewirkt auf vorteilhafte Weise einen höheren Tragekomfort, insbesondere bei einem am Körper anliegenden Inkontinenzartikel, da von der Backsheetseite her ein weicherer haptischer Eindruck des Inkontinenzartikels erreicht wird. Hierzu enthält die zweite Saugkörperlage wenig oder kein SAP. Dem Fachmann ist außerdem bekannt, dass partikelförmiges Material abrasive Kräfte ausüben kann, die das im Wesentlichen flüssigkeitsundurchlässige Backsheet, insbesondere eine flüssigkeitsundurchlässige Backsheetfolie des absorbierenden Artikels beschädigen und zu Undichtigkeit des Artikels führen können. Daher bietet die zweite Saugkörperlage auch den Vorteil, dass die Gefahr der Beschädigung der Backsheet-Folie im Herstellungsprozess oder während der Benutzung des Inkontinenzartikels verringert wird.

Es hat sich als vorteilhaft erwiesen, wenn das pH-Regulationsmittel partikelförmig ausgebildet ist.

Bevorzugt weist mindestens 50 Gewichtsprozent des pH-Regulationsmittels eine Partikelgröße von 10 bis 2000 µm, insbesondere von 50 bis 1200 µm, weiter insbesondere von 80 bis 800 µm auf.

Grundsätzlich sind auch pH-Regulationsmittel mit geringerer oder größerer Partikelgröße erhältlich und verwendbar.

Jedoch bieten die bevorzugten Partikelgrößen den Vorteil, dass sich Partikel einer solchen Größe bei Benetzung mit Körperausscheidungen wie einer Miktionsflüssigkeit langsamer auflösen als pH-Regulationsmittel geringerer Partikelgröße, so dass das pH-Regulationsmittel während der Benutzung nicht so schnell ausgewaschen wird und ein pHregulierender Effekt an einer der Haut des Benutzers zugewandten Oberseite des Inkontinenzartikels länger erhalten bleibt.

Zudem lässt sich ein pH-Regulationsmittel der bevorzugten Partikelgröße insbesondere in schnell laufenden Maschinen leichter dosieren und in den Inkontinenzartikel, insbesondere in die erste Saugkörperlage einarbeiten als beispielsweise feinpudrige pH-Regulationsmittel, da es im Herstellungsprozess zu weniger Staubentwicklung und damit verbundenem Materialverlust kommt.

Außerdem wird die prozesssichere Anordnung einer vorgesehenen Menge an pH-Regulationsmittel in einem vorgesehenen Zielbereich, insbesondere im vorderen, mittleren und hinteren Bereich positiv beeinflusst.

Andererseits bieten Partikel dieser Größe auch den Vorteil, dass sie während der Benutzung des Inkontinenzartikels haptisch weniger wahrnehmbar sind als beispielsweise Granulate mit höherer Partikelgröße, wodurch der Tragekomfort des Inkontinenzartikels verbessert wird.

Partikelförmiges pH-Regulationsmittel kann mit dem absorbierenden Material der ersten Saugkörperlage gemischt sein, also im Wesentlichen innerhalb der ersten Saugkörperlage angeordnet sein.

Bevorzugt wird das pH-Regulationsmittel jedoch auf einer körperseitigen Oberfläche der ersten Saugkörperlage angeordnet. Dadurch kann eine auf den Inkontinenzartikel auftreffende Körperflüssigkeit mit dem pH-Regulationsmittel in Kontakt treten bevor sie in der Saugkörperlage weitergeleitet oder dauerhaft gebunden wird, wodurch die Wirkung des pH-Regulationsmittels beschleunigt werden kann.

Alternativ zu partikelförmigem pH-Regulationsmittel kann das pH-Regulationsmittel auch in gelöster Form, insbesondere als wässrige oder alkoholische Lösung in den Inkontinenzartikel eingebracht sein, insbesondere durch Besprühen oder Eintauchen und optional nachfolgendem Trocknen mindestens einer, insbesondere der ersten Saugkörperlage. Als weitere Alternative kann das pH-Regulationsmittel auch Suspension oder Paste in den Inkontinenzartikel eingebracht sein

Körperausscheidungen wie Urin weisen typischerweise einen alkalischeren pH-Wert als die Haut auf und können deshalb zu Hautirritationen oder -entzündungen führen. Daher sind im Stand der Technik pH-Regulationsmittel für Inkontinenzartikel zur pH-Kontrolle von Körperausscheidungen beschrieben wie schwache Säuren oder Basen und deren jeweilige Salze oder Kombinationen davon, beispielsweise Carbonsäuren wie Citronensäure, Essigsäure, Äpfelsäure, Milchsäure und/oder deren jeweilige Salze wie beispielsweise Natriumsalze. Grundsätzlich sind alle Substanzen oder Zusammensetzungen denkbar, die eine geeignete Pufferwirkung aufweisen und bei denen eine geringe Gefahr für Hautirritationen besteht. Hierbei gilt im Rahmen der vorliegenden Erfindung, dass superabsorbierende Polymere (SAP), also insbesondere Polymer, die zumindest das 15-fache ihres Gewichts an 0,9 gewichtsprozentiger Kochsalzlösung absorbieren (gemessen nach NWSP 242.0.R2(15)), nicht als pH-Regulationsmittel zu verstehen sind.

Die Erfinder haben festgestellt, dass es diesbezüglich vorteilhaft ist, wenn das pH-Regulationsmittel Trinatriumcitrat, Mononatriumcitrat oder Dinatriumcitrat aufweist.

In einer bevorzugten Ausführungsform kann das pH-Regulationsmittel entweder Mononatriumcitrat und Trinatriumcitrat, oder Dinatriumcitrat und Trinatriumcitrat, oder Mononatriumcitrat und Dinatriumcitrat und Trinatriumcitrat aufweisen.

Durch die Auswahl oder Kombination der genannten Komponenten kann eine Feineinstellung der Pufferwirkung eines Inkontinenzartikels je nach angestrebter Benutzungsdauer oder Absorptionskapazität des Artikels erreicht werden, ohne dass wie im Falle der Verwendung von Citronensäure die Gefahr der Entstehung stark sauer reagierender Bereiche besteht.

Falls das pH-Regulationsmittel mehr als ein Citrat umfasst, weist das pH-Regulationsmittel vorzugsweise eine homogene Mischung der jeweiligen Citrate auf. Das hat den Vorteil, dass lokale Unterschiede in der Pufferwirkung des pH-Regulationsmittels vermieden werden können.

Die Begriffe Mononatriumcitrat, Dinatriumcitrat, und Trinatriumcitrat umfassen sowohl die nicht hydrierte Form als auch Hydrate der jeweiligen Substanz.

Bevorzugt besteht das pH-Regulationsmittel aus Mononatriumcitrat oder Dinatriumcitrat.

Ein Vorteil bei der Verwendung von Mononatriumcitrat ist beispielsweise, dass Mononatriumcitrat weniger hygroskopisch ist als beispielsweise Citronensäure und daher kann ein unerwünschter Eintrag von Feuchtigkeit in den Inkontinenzartikel vor der Benutzung vermieden werden. Da Mononatriumcitrat weniger hygroskopisch ist als beispielsweise Citronensäure ergibt sich als weiterer Vorteil eine bessere Rieselfähigkeit, so dass sich Mononatriumcitrat leichter dosieren lässt.

In einer bevorzugten Ausführungsform besteht das pH-Regulationsmittel aus Mononatriumcitrat.

In einer weiteren bevorzugten Ausführungsform besteht das pH-Regulationsmittel aus Dinatriumcitrat.

Durch die Verwendung nur einer Komponente als pH-Regulationsmittel ergeben sich prozesstechnische Vorteile, da keine Vermischung verschiedener Komponenten oder Aufrechterhaltung einer homogenen Durchmischung während des Herstellungsprozesses erforderlich ist. Zudem wird eine gleichmäßige Pufferwirkung über die pH-Regulationsmittel enthaltenden Bereiche des Inkontinenzartikels hinweg weiter unterstützt.

In einer bevorzugten Ausführungsform beträgt die flächenbezogene Menge an pH-Regulationsmittel in dem Kanal 5 bis 100 g/m², insbesondere 10 bis 50 g/m², weiter insbesondere 15 bis 30 g/m².

In einer bevorzugten Ausführungsform beträgt die flächenbezogene Menge an pH-Regulationsmittel in einem von einer nachfolgend näher beschriebenen Flüssigkeitsaufnahme- und Verteilerschicht überfangenen Bereich 5 bis 100 g/m², insbesondere 10 bis 50 g/m², weiter insbesondere 15 bis 30 g/m².

Die Mengenangaben des pH-Regulationsmittels sind so zu verstehen, dass sie sich auf ein wasserfreies pH-Regulationsmittel, beispielsweise nicht hydriertes Mononatriumcitrat, beziehen. Falls das pH-Regulationsmittel ein oder mehrere Hydrate oder wie nachfolgend beschrieben mehr als 5%, insbesondere mehr als 10% additive Substanzen umfasst, wird die Gesamtmenge des pH-Regulationsmittels in g/m² derart angepasst, dass die Gesamtmenge an dem enthaltenen reinen und/oder nicht hydrierten pH-Regulationsmittel der vorstehenden bevorzugten Menge entspricht.

Bevorzugt wird das Mononatriumcitrat oder Dinatriumcitrat oder Trinatriumcitrat im Wesentlichen in reiner Form verwendet, insbesondere mit einem Reinheitsgrad von mindestens 90%, weiter insbesondere mindestens 95%, weiter insbesondere mindestens 98%, weiter insbesondere mindestens 99%, weiter insbesondere 100%. Insbesondere wird das Mononatriumcitrat oder Dinatriumcitrat oder Trinatriumcitrat mit einer Reinheit verwendet, welche den Anforderungen der German Pharmaceutical Codex (DAC) oder der Commission Regulation (EU) 231/2012 genügt.

Dadurch können durch Rückstände anderer Substanzen ausgelöste Hautirritationen bei Benutzung des Inkontinenzartikels weitestgehend vermieden werden.

Es ist jedoch auch denkbar, dass das pH-Regulationsmittel eine geringe Menge einer oder mehrerer additiver Substanzen aufweist, beispielsweise Konfektionierungs- und/oder Konditionierungsmittel wie Trennmittel, Stabilisatoren, Staubbindemittel, Antibackmittel, Netzmittel, Koagulationsmittel, Antikoagulationsmittel, adhäsive oder oberflächenaktive Substanzen oder antimikrobielle Substanzen, Farb- oder Duftstoffe oder pH-Indikatoren. Solchenfalls beträgt der Anteil der einen oder mehreren anderen Substanzen an der Gesamtmenge des pH-Regulationsmittels insgesamt bevorzugt weniger als 10%, weiter bevorzugt weniger als 5%, weiter bevorzugt weniger als 2%, weiter bevorzugt weniger als 1%.

In einer bevorzugten Ausführungsform weist die erste Saugkörperlage in dem ersten Bereich zumindest eine Teilmenge des pH-Regulationsmittels auf.

Hierdurch kann eine Körperflüssigkeit, insbesondere bei einem Auftreffen einer großen Flüssigkeitsmenge, die nicht sofort am Punkt oder Bereich des Auftreffens in den Absorptionskörper, dabei insbesondere in den Kanal, aufgenommen werden kann, in vorteilhafter Weise auch in dem umgebenden ersten Bereich der ersten Saugkörperlage pH-reguliert werden.

Bevorzugt weist der Inkontinenzartikel mindestens eine Flüssigkeitsaufnahme- und Verteilerschicht (englisch: acquisition distribution layer, ADL) auf, welche zwischen dem Topsheet und der ersten Saugkörperlage angeordnet ist und wobei die ADL den Kanal bereichsweise, insbesondere vollständig überfängt.

Dadurch können Körperflüssigkeiten wie Urin rasch aufgenommen, verteilt und an die erste Saugkörperlage und/oder in den Kanal weitergeleitet werden.

Bevorzugt ist der mittlere Bereich der ersten Saugkörperlage in der orthogonalen Projektion unterhalb der ADL zu verorten. Dadurch kommt es im Benutzungszustand in vorteilhafter Weise zur Weiterleitung der aufgenommenen Körperflüssigkeit insbesondere an den pH-Regulationsmittel aufweisenden mittleren Bereich und eine rasche pH-Regulation wird ermöglicht. Nach einer Variante der Erfindung kann der vordere Bereich und/oder der hintere Bereich des Absorptionskörpers ganz oder abschnittsweise ebenfalls in der orthogonalen Projektion unterhalb der ADL zu verorten sein.

Weiter bevorzugt ist der mittlere und/oder der vordere und/oder der hintere Bereich in der orthogonalen Projektion jeweils als kreisförmig zu verorten, insbesondere mit einem Durchmesser von 2,5 cm.

Flüssigkeitsaufnahme- oder Verteilerschichten sind im Fachgebiet bereits bekannt und bestehen typischerweise aus einem Fasermaterial, insbesondere einem Vliesmaterial.

Die Fasern können natürlichen oder künstlichen Ursprungs sein und können von definierter Länge (Stapelfasern) oder kontinuierlich ("endlos") sein oder in-situ gebildet werden. Die Fasern können aus einem einzelnen Polymer oder einer Polymermischung (Einkomponentenfaser) oder aus mehr als einem einzelnen Polymer und/oder einer Polymermischung (Mehrkomponentenfaser) ausgebildet sein.

Eine Mehrkomponentenfaser weist einen Querschnitt auf, der mehr als einen einzelnen Abschnitt umfasst, wobei jeder dieser Abschnitte ein anderes Polymer oder eine andere Polymermischung umfasst. Der Begriff Mehrkomponentenfaser umfasst, ist aber nicht beschränkt auf, eine Zweikomponentenfaser. Die verschiedenen Komponenten von Mehrkomponentenfasern sind in im Wesentlichen unterschiedlichen Bereichen über den Querschnitt der Faser angeordnet und erstrecken sich kontinuierlich über die Länge der Faser. Eine Mehrkomponentenfaser kann einen Gesamtquerschnitt aufweisen, der Teilbereiche der zwei oder mehr unterschiedlichen Komponenten jeglicher Form oder Anordnung aufweist, einschließlich beispielsweise koaxialer Unterabschnitte, Kern- und Hüllenunterabschnitte, nebeneinander liegende Unterabschnitte, radiale Unterabschnitte, inselförmige Unterabschnitte, etc.

Eine Zweikomponentenfaser mit einer "Kern/Hüll-Struktur" hat einen Querschnitt, der Folgendes umfasst: zwei diskrete Abschnitte, von denen jeder aus einem Polymer oder einer Polymermischung besteht, worin das Hüllpolymer oder die Hüllpolymermischkomponente um das Kernpolymer oder die Kernpolymermischungskomponente herum angeordnet ist.

Das Flächengewicht von Vliesmaterialien wird in der Regel in Gramm pro Quadratmeter (g/m²) angegeben.

In einer bevorzugten Ausführungsform umfasst die Flüssigkeitsaufnahme- und Verteilerschicht Mehrkomponentenfasern, insbesondere Zweikomponentenfasern, weiter insbesondere Polyester aufweisende Zweikomponentenfasern, sogenannte Bico/PES-Fasern. Die Mehrkomponentenfasern, insbesondere die Zweikomponentenfasern, weisen bevorzugt einen kreisförmigen oder dreilappigen Querschnitt auf.

Bevorzugte Kombinationen von Komponenten in Zweikomponentenfasern sind Polyethylenterephthalat (PET) / Polyethylen (PE), PET / Polypropylen (PP), PET / Polyester-Copolymere (CoPET), Polymilchsäure (PLA) / Polylactid-Copolymere (COPLA), PLA / PE und PLA / PP.

Als eine Alternative ist es im Fachgebiet bekannt, ein Material aus chemisch modifizierten Zellulosefasern, beispielsweise quervernetzten Zellulosefasern, als ADL zu verwenden.

Die Flüssigkeitsaufnahme- und Verteilerschicht kann auch andere Materialien wie perforierte Folien oder Schäume oder dergleichen umfassen oder daraus bestehen.

Die Flüssigkeitsaufnahme- und Verteilerschicht kann den Absorptionskörper im Wesentlichen vollständig oder nur teilweise überfangen, mithin im Wesentlichen dieselbe flächenhafte Erstreckung oder eine zumindest bereichsweise geringere Erstreckung als die erste Saugkörperlage und/oder die zweite Saugkörperlage des Absorptionskörpers in Längs- und/oder Querrichtung des plan aufliegenden Inkontinenzartikels aufweisen. Bevorzugt überfängt die Flüssigkeitsaufnahme- und Verteilerschicht die erste Saugkörperlage und/oder die zweite Saugkörperlage des Absorptionskörpers zu 5-100%, insbesondere zu 10-90%, weiter insbesondere zu 15-80%, weiter insbesondere zu 15-70% ihrer flächenhaften Erstreckung.

Vorzugsweise ist die Flüssigkeitsaufnahme- und Verteilerschicht zumindest in einem Bereich angeordnet in welchem die Miktionsflüssigkeit in Gebrauch auf den Inkontinenzartikel trifft. Insbesondere erstreckt sich die Flüssigkeitsaufnahme- und Verteilerschicht über und im Bereich einer Quermittelachse des Absorptionskörpers.

In einer bevorzugten Ausführungsform ist das pH-Regulationsmittel im Wesentlichen zwischen der Flüssigkeitsaufnahme- und Verteilerschicht und der ersten Saugkörperlage angeordnet.

Insbesondere sind zumindest 50 Gewichtsprozent, insbesondere mindestens 60 Gewichtsprozent, weiter insbesondere 70 Gewichtsprozent, weiter insbesondere zumindest 80 Gewichtsprozent des pH-Regulationsmittels zwischen der Flüssigkeitsaufnahme- und Verteilerschicht und der ersten Saugkörperlage angeordnet.

Daraus ergibt sich in vorteilhafter Weise, dass aufgenommene Körperflüssigkeiten bereits nach Passieren der Flüssigkeitsaufnahme- und Verteilerschicht mit dem pH-Regulationsmittel in Kontakt treten und der pH-Wert rasch reguliert werden kann.

Die Gesamtmenge an pH-Regulationsmittel pro Inkontinenzartikel, der insbesondere zur Benutzung durch Erwachsene vorgesehen ist, beträgt vorzugsweise 0,20 bis 3,00 g, weiter vorzugsweise 0,30 bis 2,50 g, weiter vorzugsweise 0,40 bis 2,00 g, weiter vorzugsweise 0,50 bis 1,50 g, weiter vorzugsweise 0,50 bis 1,00 g, weiter vorzugsweise 0,50 bis 0,80 g.

In einer bevorzugten Ausführungsform erhält der Inkontinenzartikel über mindestens zwei, insbesondere mindestens drei Miktionsereignisse hinweg, einen hautfreundlichen pH-Wert aufrecht.

Dadurch wird der Tragekomfort auch über einen längeren Benutzungszeitraum wie beispielsweise während der Nachtruhe verbessert.

Weiter bevorzugt beträgt der pH-Wert auf einer der Haut zugewandten Oberseite des Inkontinenzartikels einen Wert von 4,0-6,5, insbesondere 4,2-6,2, weiter insbesondere 4,3-6,0, weiter insbesondere 4,5-5,8, weiter insbesondere 4,7-5,7.

In einer weiteren bevorzugten Ausführungsform ist der Kanal der ersten Saugkörperlage im Wesentlichen frei von SAP.

Das bietet den Vorteil, dass die in den Kanal aufgenommene Körperflüssigkeit nicht im Kanal vorzeitig dauerhaft gebunden wird, sondern ungehindert entlang des Kanals weitergeleitet und dadurch gleichmäßiger im Absorptionskörper verteilt werden kann. Insbesondere kann eine vorzeitige Bindung der aufgenommenen Körperflüssigkeit mittels SAP im Kanal die pH-Regulation der Körperflüssigkeit behindern oder zumindest verzögern.

Bevorzugt weist die zweite Saugkörperlage weniger als 20 Gewichtsprozent, insbesondere weniger als 15 Gewichtsprozent, weiter insbesondere weniger als 10 Gewichtsprozent, weiter insbesondere weniger als 5 Gewichtsprozent des pH-Regulationsmittels auf. Weiter insbesondere ist die zweite Saugkörperlage frei von pH-Regulationsmittel.

Dadurch wird auf vorteilhafte Weise ein höherer Tragekomfort erreicht, insbesondere bei einem am Körper anliegenden Inkontinenzartikel, da von der Backsheetseite her ein weicherer haptischer Eindruck des Inkontinenzartikels erreicht wird. Wie vorgehend beschrieben kann partikelförmiges Material abrasive Kräfte ausüben, die das im Wesentlichen flüssigkeitsundurchlässige Backsheet, insbesondere eine flüssigkeitsundurchlässige Backsheetfolie des absorbierenden Artikels beschädigen und zu Undichtigkeit des Artikels führen können. Daher ist ein weiterer Vorteil, dass die Gefahr der Beschädigung der Backsheet-Folie im Herstellungsprozess oder während der Benutzung des Inkontinenzartikels verringert wird.

Bei dem Inkontinenzartikel kann es sich um unterschiedliche Ausführungsformen handeln wie beispielsweise Inkontinenzvorlagen, Inkontinenzeinlagen, Slipeinlagen, Inkontinenzwindeln offenen Typs, Inkontinenzwindeln geschlossenen Typs oder auch Krankenunterlagen.

Je nach Typ oder Ausführungsform kann der Inkontinenzartikel ein oder mehrere weitere im Stand der Technik hinlänglich bekannte Merkmale aufweisen wie beispielsweise Seitenteile, Verschlusssysteme, Flügel, Elastifizierungen, Auslaufschutzsysteme (z.B. Cuffs), Nässeanzeiger, Kennzeichnungsmittel, hautpflegende oder geruchsregulierende Substanzen oder Zusammensetzungen, Befestigungsmittel (z.B. Klebstoff, Haken) sowie andere dem Fachmann bekannte oder sinnvoll erscheinende Merkmale.

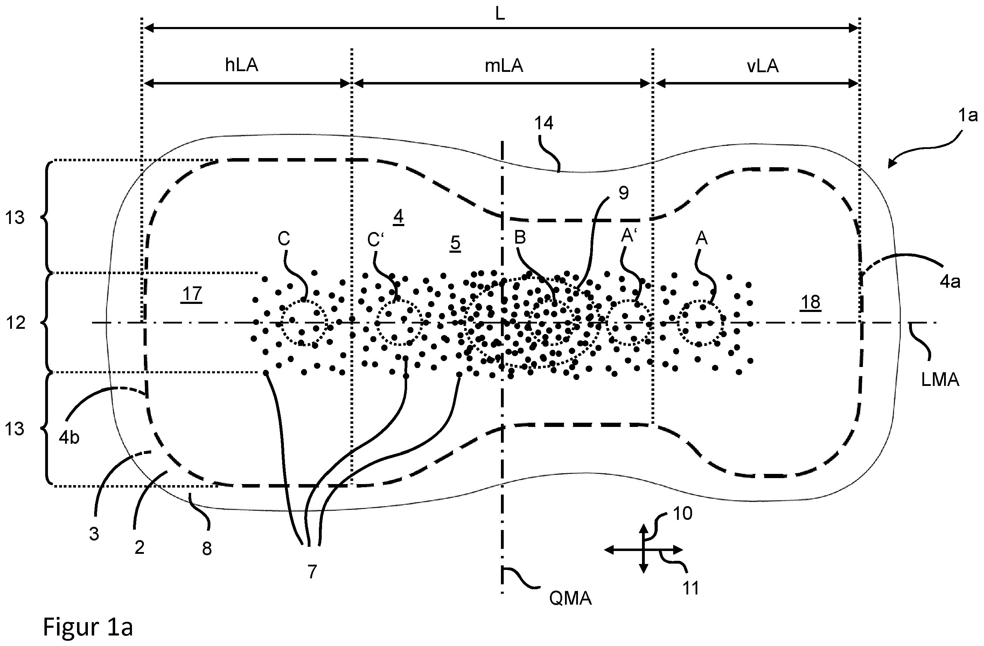

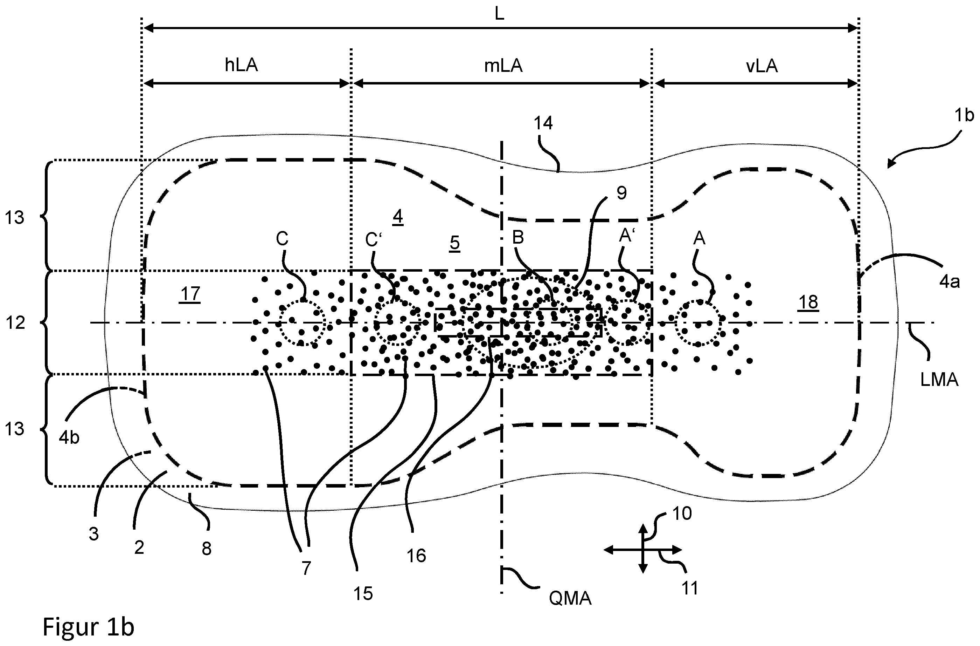

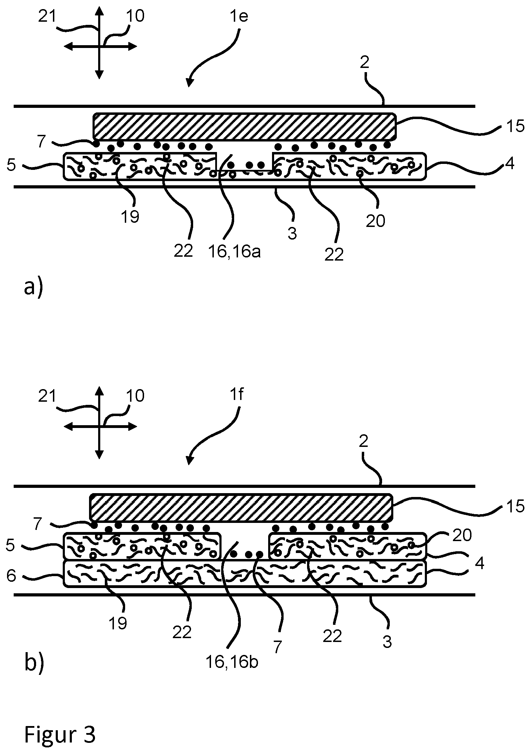

Weitere Merkmale, Einzelheiten und Vorteile der Erfindung ergeben sich aus den beigefügten Patentansprüchen und aus der zeichnerischen Darstellung und nachfolgenden Beschreibung bevorzugter Ausführungsformen der Erfindung und Beispielen. In der Zeichnung zeigt:
**Figur 1a**
   schematische Darstellung in Draufsicht eines Inkontinenzartikels
**Figur 1b**
   schematische Darstellung in Draufsicht eines Inkontinenzartikels analog Figur 1a, zusätzlich ADL und Kanal aufweisend
**Figur 2a**
   schematisch, einen Querschnitt durch einen Inkontinenzartikel mit einer ersten Saugkörperlage, ADL und pH-Regulationsmittel
**Figur 2b**
   schematisch, einen Querschnitt durch einen Inkontinenzartikel mit einer ersten Saugkörperlage mit SAP und einer zweiten Saugkörperlage, einer ADL und pH-Regulationsmittel
**Figur 3a**
   schematisch, einen Querschnitt durch einen Inkontinenzartikel analog Figur 1b, mit einer ersten Saugkörperlage und Kanal
**Figur 3b**
   schematisch, einen Querschnitt durch einen Inkontinenzartikel analog Figur 3a mit einer ersten und einer zusätzlichen zweiten Saugkörperlage und Kanal
**Figur 4**
   schematische Darstellung in Draufsicht einer Inkontinenzwindel offenen Typs mit Verschlusselementen

**Figur 1a** zeigt schematisch, nicht maßstäblich, einen erfindungsgemäßen Inkontinenzartikel 1a mit einem zumindest bereichsweise flüssigkeitsdurchlässigen Topsheet 2, einem im Wesentlichen flüssigkeitsundurchlässigen Backsheet 3 und einen zwischen dem Topsheet 2 und dem Backsheet 3 angeordneten Absorptionskörper 4, wobei der Absorptionskörper 4 eine Quermittelachse QMA, eine Längsmittelachse LMA und eine erste Saugkörperlage 5 aufweist.

Die erste Saugkörperlage 5 weist ein pH-Regulationsmittel 7, beispielsweise partikelförmiges Mononatriumcitrat oder Trinatriumcitrat oder Dinatriumcitrat, auf, hier punktförmig dargestellt. Das pH-Regulationsmittel 7 ist ungleichförmig in die erste Saugkörperlage 5 eingebracht, so dass ein mittlerer Bereich B des Absorptionskörpers 4 und ein vorderer Bereich A des Absorptionskörpers 4 und ein hinterer Bereich C des Absorptionskörpers 4 pH-Regulationsmittel 7 enthalten, derart, dass eine flächenbezogene Menge des pH-Regulationsmittels 7 in dem mittleren Bereich B des Absorptionskörpers 4 in g/m² größer ist als in dem vorderen A und/oder dem hinteren Bereich C des Absorptionskörpers 4 (in Figur 1a schematisch verdeutlicht durch eine höhere Anzahl und Dichte des punktförmig dargestellten pH-Regulationsmittels 7 im mittleren Bereich B).

Der Absorptionskörper 4 weist eine im Benutzungszustand bauchseitig zu liegen kommende vordere Querkante 4a und eine im Benutzungszustand rückenseitig zu liegen kommende hintere Querkante 4b auf, die sich jeweils in einer Querrichtung 10 des Absorptionskörpers, welche hier einer Querrichtung des Inkontinenzartikels entspricht, erstreckt. Die vordere 4a und die hintere Querkante 4b des Absorptionskörpers 4 verlaufen hier bereichsweise gerade. Alternativ können die vordere 4a und/oder die hintere Querkante 4b des Absorptionskörpers auch vollständig gerade, abgewinkelt, gekrümmt, gebogen, wellenförmig oder mit einer anderen dem Fachmann als geeignet erscheinenden Linienführung verlaufen.

Ein vorderer A, mittlerer B und hinterer Bereich C sind auf der Längsmittelachse LMA des Absorptionskörpers 4 zu verorten. Ein vorderer Bereich A ist dabei näher an der vorderen Querkante 4a des Absorptionskörpers 4 angeordnet als ein mittlerer B und ein hinterer Bereich C. Der hintere Bereich C ist näher an der hinteren Querkante 4b des Absorptionskörpers 4 angeordnet als der mittlere B und vordere Bereich A. Der mittlere Bereich B ist im dargestellten Beispiel in dem Miktionsbereich 9 zu verorten, in welchem im Benutzungszustand mit hoher Wahrscheinlichkeit Körperflüssigkeiten wie Urin auf den Inkontinenzartikel 1a auftreffen.

Eine flächenbezogene Menge des pH-Regulationsmittels 7 ist in einem mittleren Längsabschnitt mLA des Absorptionskörpers 4 in g/m² größer als in einem vorderen vLA und/oder einem hinteren Längsabschnitt hLA des Absorptionskörpers 4. Die Länge des mittleren Längsabschnitts mLA kann 30-65% einer Gesamtlängserstreckung L des Absorptionskörpers betragen. Figur 1a veranschaulicht nur beispielhaft die Verortung eines mittleren Längsabschnitts mLA mit einer Länge von 43% der Gesamtlängserstreckung L des Absorptionskörpers. Sowohl ein beliebiger mittlerer mLA als auch ein beliebiger vorderer vLA und ein beliebiger hinterer Längsabschnitt hLA des Absorptionskörpers 4 weisen in diesem Beispiel pH-Regulationsmittel 7 auf.

Das pH-Regulationsmittel 7 ist in diesem Beispiel beidseitig der Längsmittelachse LMA des Absorptionskörpers 4, dabei in einem in Querrichtung 10 zentralen Bereich 12 des Absorptionskörpers 4 angeordnet, wobei in Querrichtung 10 periphere Bereiche 13 des Absorptionskörpers 4 frei von pH-Regulationsmittel 7 sind. Dadurch wird das pH-Regulationsmittel 7 dort fokussiert, wo Körperflüssigkeiten typischerweise vorrangig auf den Inkontinenzartikel 1a auftreffen, nämlich im zentralen Bereich 12. Das pH-Regulationsmittel 7 ist dabei auch in dem Miktionsbereich 9 (auch als Miktionspunkt bezeichnet) angeordnet, in welchem eine Miktionsflüssigkeit im Benutzungszustand mit hoher Wahrscheinlichkeit auf den Inkontinenzartikel 1a trifft. Es ist aber auch möglich, das pH-Regulationsmittel 7 sowohl im zentralen Bereich 12 als auch in den peripheren Bereichen 13 anzuordnen.

Der vordere vLA und der hintere Längsabschnitt hLA des Absorptionskörpers 4 weisen in dieser bevorzugten Ausführungsform in an die vordere 4a oder hintere Querkante 4b angrenzenden Teilabschnitten 17,18 kein pH-Regulationsmittel 7 auf. In einer alternativen Ausführungsform kann das pH-Regulationsmittel 7 jedoch über die gesamte Längserstreckung L des Absorptionskörpers 4 hinweg angeordnet sein. In einer weiteren alternativen Ausführungsform kann das pH-Regulationsmittel 7 nur innerhalb des mittleren Längsabschnitts mLA des Absorptionskörpers 4 angeordnet sein.

Der Absorptionskörper 4 und ein gemeinsam durch das Topsheet 2 und das Backsheet 3 gebildetes Chassis 8 sind in diesem Ausführungsbeispiel anatomisch ausgebildet. Sie können jedoch auch rechteckig, dreieckig, oval, T-förmig, sanduhrförmig, anatomisch, asymmetrisch oder in einer anderen dem Fachmann als geeignet erscheinenden Form ausgebildet sein.

Der mittlere Längsabschnitt mLA des Absorptionskörpers 4 ist in einem Schrittbereich 14 des Inkontinenzartikels 1a, mithin sich über den Miktionsbereich 9 erstreckend, angeordnet und erstreckt sich zudem über die Quermittelachse QMA des Absorptionskörpers 4 hinweg. Der mittlere Längsabschnitt mLA des Absorptionskörpers 4 ist dabei symmetrisch zu der Quermittelachse QMA des Absorptionskörpers 4 angeordnet.

Der vordere Längsabschnitt vLA des Absorptionskörpers 4 schließt sich in Längsrichtung 11 an den mittleren Längsabschnitt mLA in Richtung der vorderen Querkante 4a des Absorptionskörpers 4 an und erstreckt sich bis zu der vorderen Querkante 4a des Absorptionskörpers 4.

Der hintere Längsabschnitt hLA des Absorptionskörpers 4 schließt sich in Längsrichtung 11 an den mittleren Längsabschnitt mLA in Richtung der hinteren Querkante 4b des Absorptionskörpers 4 an und erstreckt sich bis zu der hinteren Querkante 4b des Absorptionskörpers 4. Die Grenzen zwischen mittlerem mLA und vorderem Längsabschnitt vLA und zwischen mittlerem mLA und hinterem Längsabschnitt hLA verlaufen jeweils geradlinig in Querrichtung 10 des Inkontinenzartikels 1a und orthogonal zu der Längsmittelachse LMA des Absorptionskörpers 4.

In diesem bevorzugten Beispiel entspricht die Längsmittelachse LMA des Absorptionskörpers 4 einer Längsmittelachse des Inkontinenzartikels 1a und einer Längsmittelachse der ersten Saugkörperlage 5. Ebenso entspricht hier die Längsrichtung 11 des Absorptionskörpers 4 einer Längsrichtung des Inkontinenzartikels 1a und einer Längsrichtung der ersten Saugkörperlage 5.

Weiterhin entspricht die Quermittelachse QMA des Absorptionskörpers 4 einer Quermittelachse des Inkontinenzartikels 1a und einer Quermittelachse der ersten Saugkörperlage 5. Ebenso entspricht hier die Querrichtung 10 des Absorptionskörpers 4 einer Querrichtung des Inkontinenzartikels 1a und einer Querrichtung der ersten Saugkörperlage 5.

In dem dargestellten Beispiel ist ein mittlerer Bereich B des Absorptionskörpers 4 in dem mittleren Längsabschnitt mLA des Absorptionskörpers 4 zu verorten. Ein vorderer Bereich A des Absorptionskörpers 4 kann in dem vorderen Längsabschnitt vLA des Absorptionskörpers 4 verortet werden. Ein hinterer Bereich C des Absorptionskörpers 4 kann in dem hinteren Längsabschnitt hLA des Absorptionskörpers 4 verortet werden. Außerdem können zusätzlich ein vorderer Bereich A' und ein hinterer Bereich C' des Absorptionskörpers 4 in dem mittleren Längsabschnitt mLA des Absorptionskörpers 4 verortet werden.

Die erste Saugkörperlage 5 ist in diesem bevorzugten Ausführungsbeispiel deckungsgleich, also in einer Längsrichtung 11 des Absorptionskörpers 4 und in einer Querrichtung 10 des Absorptionskörpers 4 gleich weit erstreckt wie der Absorptionskörper 4. Daher entspricht die Gesamtlängserstreckung L des Absorptionskörpers einer Gesamtlängserstreckung der ersten Saugkörperlage 5 und eine maximale Quererstreckung der ersten Saugkörperlage 5 entspricht einer maximalen Quererstreckung des Absorptionskörpers 4. Alternativ können die erste Saugkörperlage 5 und der Absorptionskörper 4 in Querrichtung 10 oder Längsrichtung 11 des Absorptionskörpers unterschiedlich weit erstreckt sein.

Der in Figur 1a dargestellte Inkontinenzartikel kann zusätzliche optionale Merkmale aufweisen, die im Stand der Technik hinreichend beschrieben sind, wie beispielsweise Elastifizierungsmittel, Flüssigkeitsbarrieren (z.B. Cuffs) oder Befestigungsmittel.

**Figur 1b** zeigt schematisch, nicht maßstäblich, mit Bezug zum Inkontinenzartikel 1a in Figur 1a einen weiteren Inkontinenzartikel 1b, der zusätzlich eine Flüssigkeitsaufnahme- und Verteilerschicht (ADL) 15 und einen Kanal 16 aufweist. In diesem Ausführungsbeispiel weist die ADL 15 eine Erstreckung in der Längsrichtung 11 des Absorptionskörpers 4 auf, die der Längserstreckung des mittleren Längsabschnitts mLA entspricht. Die ADL 15 überfängt in diesem Beispiel 18 % einer Gesamtfläche des Absorptionskörpers 4. Es wurde festgestellt, dass dieser Flächenanteil auf vorteilhafte Weise gleichzeitig eine effiziente Flüssigkeitsaufnahme und -Verteilung bietet und kostengünstig ist. Eine geeignete ADL 15 kann jedoch zwischen 5 und 100 % der Gesamtfläche des Absorptionskörpers 4 überfangen. In diesem Ausführungsbeispiel ist die ADL 15 symmetrisch zur Quermittelachse QMA des Absorptionskörpers 4 angeordnet. Die ADL 15 kann jedoch auch asymmetrisch zur Quermittelachse QMA des Absorptionskörpers 4 oder zu einer Quermittelachse des Inkontinenzartikels angeordnet sein, mithin näher bei der vorderen Querkante 4a oder der hinteren Querkante 4b des Absorptionskörpers als bei der jeweiligen anderen hinteren 4b oder vorderen Querkante 4a des Absorptionskörpers 4 angeordnet sein.

Sowohl die ADL 15 als auch der Kanal 16 erstrecken sich über den Miktionsbereich 9. Ein mittlerer Bereich B des Absorptionskörpers 4 ist in diesem Beispiel in der orthogonalen Projektion unterhalb der ADL 15 zu verorten. Ein vorderer A und ein hinterer Bereich C des Absorptionskörpers 4 sind in der orthogonalen Projektion außerhalb der ADL 15 zu verorten. Der mittlere Bereich B des Absorptionskörpers 4 ist in diesem Beispiel außerdem im Bereich des Kanals 16 zu verorten. Die flächenbezogene Menge an pH-Regulationsmittel 7 des mittleren Bereichs B entspricht in diesem Ausführungsbeispiel im Wesentlichen der flächenbezogenen Menge an pH-Regulationsmittel in dem Kanal 16.

Der Kanal 16 ist parallel zu und auf der Längsmittelachse LMA des Absorptionskörpers 4 und des Inkontinenzartikels 1b angeordnet. Weiter ist der Kanal 16 symmetrisch zur Längsmittelachse LMA des Absorptionskörpers 4 und Inkontinenzartikels 1b ausgebildet. Auf vorteilhafte Weise ist der Kanal 16 hier in Querrichtung 10 des Absorptionskörpers 4 zentriert angeordnet und erstreckt sich symmetrisch über die Quermittelachse QMA des Absorptionskörpers 4 hinweg.

Das Beispiel zeigt einen bevorzugten Kanal 16, der von der hinteren Querkante 4b des Absorptionskörpers 4 weiter entfernt ist als von der vorderen Querkante 4a des Absorptionskörpers 4. Jedoch kann den Kanal 16 auch gleich weit von der vorderen 4a und hinteren Querkante 4b des Absorptionskörpers 4 entfernt oder weiter von der vorderen 4a als von der hinteren Querkante 4b des Absorptionskörpers 4 entfernt angeordnet sein.

In diesem Beispiel ist der Kanal 16 rechteckig ausgebildet und erstreckt sich geradlinig in der Längsrichtung 11 des Absorptionskörpers und Inkontinenzartikels. Jedoch sind auch wellenförmige, gezackte oder bogenförmige oder jede andere Kanalform denkbar, die der Fachmann für sinnvoll erachtet. Unterschiedliche weitere Kanalformen wie beispielsweise mandelförmig, knochenförmig, T-förmig, dreieckig, oval, sternförmig oder verzweigt sind im Stand der Technik hinreichend bekannt.

Der Kanal 16 in diesem Beispiel weist auf vorteilhafte Weise eine größere Erstreckung in der Längsrichtung 11 des Absorptionskörpers 4 auf als in der Querrichtung 10.

Der Kanal 16 ist vollständig von der ADL 15 überfangen und weist eine Teilmenge des pH-Regulationsmittels 7 auf. Dabei kann eine im Benutzungszustand in den Kanal 16 geleitete Körperflüssigkeit rasch auf das pH-Regulationsmittel 7 treffen.

Ein einzelner zentraler Kanal 16 wie hier in der Figur 1b dargestellt wirkt sich vorteilhaft auf eine Stabilität und Form des Inkontinenzartikels 1b im Benutzungszustand aus. Jedoch ist auch denkbar, mehr als einen Kanal, insbesondere 2 Kanäle vorzusehen.

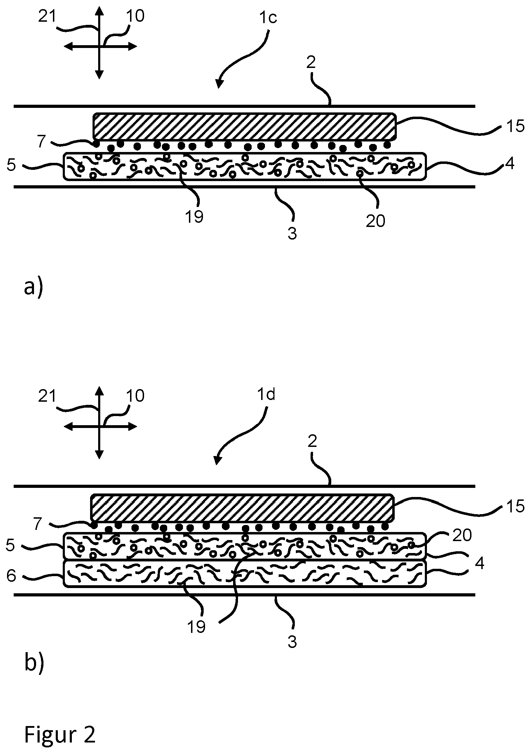

Die **Figur 2a** zeigt in beispielhafter Weise schematisch, nicht maßstäblich, einen Querschnitt entlang der Quermittelachse QMA eines Inkontinenzartikels. In diesem Inkontinenzartikel 1c ist ein mittlerer Bereich B des Absorptionskörpers 4 auf der Quermittelachse QMA zu verorten. Der Inkontinenzartikel 1c weist die in **Figur 1a** dargestellten Merkmale auf. Zusätzlich weist der Inkontinenzartikel 1c in dieser bevorzugten Ausführungsform eine Flüssigkeitsaufnahme- und Verteilerschicht (ADL) 15 auf.

Der Inkontinenzartikel 1c weist ein flüssigkeitsdurchlässiges Topsheet 2, ein im Wesentlichen (mithin in Gebrauch) flüssigkeitsundurchlässiges Backsheet 3, und einen dazwischen angeordneten Absorptionskörper 4 auf. Der Absorptionskörper 4 des Inkontinenzartikels 1c weist eine erste Saugkörperlage 5 mit Fasermaterial 19, wie beispielsweise Zellstofffasern oder Kunststofffasern, und partikelförmiges SAP 20 auf. Das SAP 20 ist im gezeigten Fall gleichmäßig verteilt in der ersten Saugkörpereinlage 5 angeordnet. Die ADL 15 ist zwischen der ersten Saugkörperlage 5 und dem Topsheet 2 angeordnet.

In dieser bevorzugten Ausführungsform ist das pH-Regulationsmittel 7 zwischen der ersten Saugkörperlage 5 und der ADL 15 angeordnet, mithin auf einer dem Topsheet 2 zugewandten Oberfläche der ersten Saugkörperlage 5. Es ist vorteilhaft, wenn von der Topsheetseite eindringende Körperflüssigkeiten zunächst auf das pH-Regulierungsmittel 7 treffen bevor sie durch SAP 20 dauerhaft gebunden werden, da so eine effektivere pH-Wert-Kontrolle erfolgen kann.

Alternativ kann das pH-Regulationsmittel 7 auch im Inneren der ersten Saugkörperlage 5, dabei entlang der Dickenrichtung 21 der ersten Saugkörperlage 5 gleichförmig oder ungleichförmig angeordnet sein.

Die **Figur 2b** zeigt schematisch, nicht maßstäblich, einen Querschnitt entlang der Quermittelachse QMA eines weiteren bevorzugten Inkontinenzartikels 1d. In diesem Inkontinenzartikel 1d ist ein mittlerer Bereich B des Absorptionskörpers 4 auf der Quermittelachse QMA zu verorten. Der Inkontinenzartikel 1c weist die in **Figur 2a** dargestellten Merkmale auf. Zusätzlich weist der Inkontinenzartikel 1c in dieser bevorzugten Ausführungsform neben der Flüssigkeitsaufnahme- und Verteilerschicht (ADL) 15 noch eine zweite Saugkörperlage 6 auf.

Die zweite Saugkörperlage 6 ist unterhalb der ersten Saugkörperlage 5 angeordnet, also zwischen erster Saugkörperlage 5 und dem Backsheet 3. In dieser bevorzugten Ausführungsform weist die zweite Saugkörperlage 6 kein SAP auf. Weiterhin weist die zweite Saugkörperlage 6 auch kein pH-Regulierungsmittel 7 auf. Dadurch wird der Tragekomfort des Inkontinenzartikels von der Backsheetseite verbessert und die Gefahr von Beschädigungen des Backsheets durch Partikel verringert. Alternativ können geringe Mengen an SAP und/oder pH-Regulierungsmittel in der zweiten Saugkörperlage 6 vorgesehen sein.

Die zweite Saugkörperlage 6 besteht im Wesentlichen aus Fasermaterial 19, wie beispielsweise Zellstofffasern oder Kunststofffasern.

Die **Figur 3a** zeigt schematisch, nicht maßstäblich, einen Querschnitt entlang der Quermittelachse QMA eines weiteren bevorzugten Inkontinenzartikels 1e. In diesem Inkontinenzartikel 1e ist eine mittlerer Bereich B des Absorptionskörpers 4 auf der Quermittelachse QMA zu verorten. Der Inkontinenzartikel 1e weist die in **Figur 2a** dargestellten Merkmale auf. Zusätzlich weist der Inkontinenzartikel 1e in dieser bevorzugten Ausführungsform neben der Flüssigkeitsaufnahme- und Verteilerschicht (ADL) 15 noch einen Kanal 16 auf.

Der Kanal 16 erstreckt sich in diesem Beispiel nicht vollständig durch die erste Saugkörperlage 5 hindurch. Dabei kann der Kanal 16 beispielsweise mittels Prägen oder Komprimieren der ersten Saugkörperlage 5 ausgebildet sein.

Dabei ist der Kanal 16 im Wesentlichen frei von absorbierenden Materialien, nämlich SAP 20 und Fasermaterial 19. Der Kanal 16 kann alternativ, wie nachfolgend in Bezug zu **Figur 3b** näher beschrieben, als eine sich durch die erste Saugkörperlage 5 in Dickenrichtung 21 hindurcherstreckende Ausnehmung gebildet sein.

In dieser bevorzugten Ausführungsform weist die erste Saugkörperlage 5 einen einzigen Kanal 16 auf, der auf einer Längsmittelachse LMA des Absorptionskörpers 4 angeordnet ist. Weiter ist der Kanal 16 symmetrisch zur Längsmittelachse LMA des Absorptionskörpers 4 ausgebildet. Der Kanal 16 ist auf vorteilhafte Weise in Querrichtung 10 des Absorptionskörpers 4 zentriert angeordnet.

Alternativ kann die erste Saugkörperlage mehr als einen Kanal, insbesondere 2-5, weiter insbesondere genau 2 Kanäle aufweisen.

Die erste Saugkörperlage 5 weist einen ersten Bereich 22 auf, der an den Kanal 16 angrenzt und der die gesamte Menge des SAP 20 der ersten Saugkörperlage 5 enthält.

Die **Figur 3b** zeigt schematisch, nicht maßstäblich, einen Querschnitt entlang der Quermittelachse QMA eines weiteren bevorzugten Inkontinenzartikels. In diesem Inkontinenzartikel 1f ist der mittlere Bereich B des Absorptionskörpers 4 auf der Quermittelachse QMA zu verorten.

Der Inkontinenzartikel 1f weist die in **Figur 3a** dargestellten Merkmale auf. Zusätzlich weist der Inkontinenzartikel 1f in dieser bevorzugten Ausführungsform neben der Flüssigkeitsaufnahme- und Verteilerschicht (ADL) 15 und einem Kanal 16 noch eine zweite Saugkörperlage 6 auf. Die Merkmale der zweiten Saugkörperlage 6 sind mit Bezug zu **Figur 2b** näher beschrieben. Der Kanal 16 ist in diesem bevorzugten Ausführungsbeispiel als eine sich durch die erste Saugkörperlage 5 in Dickenrichtung 21 hindurcherstreckende Ausnehmung 16b gebildet. Der Kanal 16 kann ebenso wie mit Bezug zu **Figur 3a** näher beschrieben als eine sich in der ersten Saugkörperlage 5 in Dickenrichtung 21 erstreckende Ausnehmung 16a gebildet sein. Als eine alternative Ausführungsform kann der Kanal 16 auch mittels Prägen oder Komprimieren der ersten Saugkörperlage 5 ausgebildet sein.

In den in **Figuren 1a**,**b**, **2a**,**b** und **3a**,**b** dargestellten Ausführungsbeispielen umfasst das pH-Regulationsmittel 7 partikelförmiges Mononatriumcitrat, Typ "Fine Granular F3500" von Jungbunzlauer. Dieses Mononatriumcitrat weist zu 59 Gewichtsprozent eine Partikelgröße von 2 bis 355 µm und eine Reinheit von mindestens 99 % auf.

In den in **Figuren 1a**,**b**, **2a**,**b** und **3a**,**b** dargestellten Ausführungsbeispielen ist das SAP 20 partikelförmig ausgebildet. In einer denkbaren Variante kann das SAP faserförmig oder blatt- oder schaumförmig ausgebildet sein.

Die ADL 15 überfängt die erste Saugkörperlage 5 des Absorptionskörpers 4 nur teilweise und weist vorliegend eine geringere Erstreckung als die erste Saugkörperlage 5 **(****Figuren 2a**,**b** und **3a**,**b**) und gegebenenfalls die zweite Saugkörperlage 6 **(****Figuren 2b** **und** **3b****)** auf. Alternativ kann die die ADL 15 die erste Saugkörperlage 5 und/oder die zweite Saugkörperlage 6 vollständig überfangen.

Die erste Saugkörperlage 5 ist in diesen Fällen (**Figuren 2b** und **3b**) in der Querrichtung 10 im Wesentlichen gleich erstreckt wie die zweite Saugkörperlage 6 des Absorptionskörpers 4. Die erste Saugkörperlage 5 und die zweite Saugkörperlage 6 können aber auch unterschiedlich erstreckt sein.

Der erste Bereich 22 der ersten Saugkörperlage 5 und die zweite Saugkörperlage 6 sind in den beschriebenen bevorzugten Ausführungsbeispielen als gleichförmig dicke Lagen, also mit einer gleichförmigen Erstreckung in der Dickenrichtung 21, dargestellt. Gleichwohl kann der erste Bereich 22 der ersten Saugkörperlage 5 und/oder die zweite Saugkörperlage 6 ein Dickenprofil aufweisen.

Die in **Figuren 2a, 2b****,** **3a** und **3b** dargestellten Ausführungsformen sind so zu verstehen, dass sie ein oder mehrere weitere typische Merkmale von Inkontinenzartikeln aufweisen können, insbesondere dass sie als verschiedene Typen von Inkontinenzartikeln ausgebildet sein können wie beispielsweise Inkontinenzvorlagen, Inkontinenzeinlagen, Slipeinlagen, Windeln des offenen oder des geschlossenen Typs oder Krankenunterlagen.

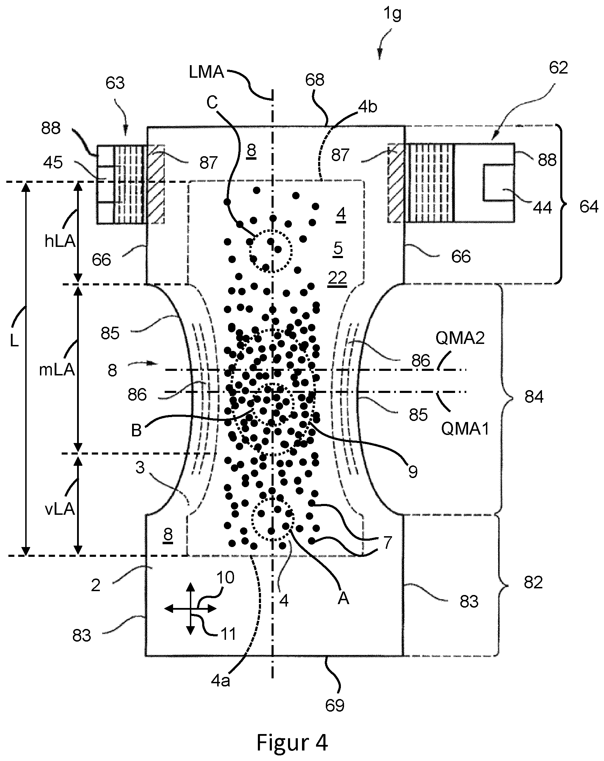

Die **Figur 4** zeigt, nicht maßstäblich, sondern schematisch einen insgesamt mit dem Bezugszeichen 1g bezeichneten erfindungsgemäßen Inkontinenzartikel, beispielhaft eine Inkontinenzwindel offenen Typs mit Verschlusselementen in der sogenannten T-Form für Erwachsene. Der Inkontinenzartikel 1g umfasst ein gemeinsam durch das Topsheet 2 und das Backsheet 3 gebildetes Chassis 8 mit einem Körperflüssigkeiten absorbierenden Absorptionskörper 4. Der Absorptionskörper 4 umfasst in diesem Ausführungsbeispiel eine erste Saugkörperlage 5, die SAP 20 (in **Figur 4** nicht gezeigt) enthält. Das SAP 20 ist dabei gleichmäßig verteilt in der ersten Saugkörperlage 5 angeordnet, wie mit Bezug zu **Figur 2a** bereits ausgeführt.

Optional kann der Inkontinenzartikel auch eine wie mit Bezug zu **Figur 2b** näher beschriebene und dort mit Bezugszeichen 6 versehene zweite Saugkörperlage, die frei von pH-Regulationsmittel ist, und eine wie mit Bezug zu **Figuren 1b****,** **2a**,**b** und **3a**,**b** näher beschriebene und dort mit Bezugszeichen 15 bezeichnete ADL umfassen.

Der Inkontinenzartikel 1g enthält ein pH-Regulationsmittel 7, bevorzugt umfassend Mononatriumcitrat oder Trinatriumcitrat oder Dinatriumcitrat oder bestehend aus Mononatriumcitrat oder Dinatriumcitrat, das auf einer dem Topsheet 2 zugewandten Oberseite der ersten Saugkörperlage 5 des Absorptionskörpers 4 angeordnet ist.

Das pH-Regulationsmittel 7 ist ungleichförmig in die erste Saugkörperlage 5 eingebracht, so dass ein mittlerer Bereich B des Absorptionskörpers 4 und ein vorderer Bereich A des Absorptionskörpers 4 und ein hinterer Bereich C des Absorptionskörpers 4 pH-Regulationsmittel 7 enthalten, derart, dass eine flächenbezogene Menge des pH-Regulationsmittels 7 in dem mittleren Bereich B des Absorptionskörpers 4 in g/m² größer ist als in dem vorderen A und dem hinteren Bereich C des Absorptionskörpers 4 (verdeutlicht durch eine höhere Anzahl und Dichte des punktförmig dargestellten pH-Regulationsmittels 7 im mittleren Bereich B).

Der Absorptionskörper 4 weist eine im Benutzungszustand bauchseitig zu liegen kommende vordere Querkante 4a und eine im Benutzungszustand rückenseitig zu liegen kommende hintere Querkante 4b auf, die sich jeweils in einer Querrichtung 10 des Absorptionskörpers, welche hier einer Querrichtung des Inkontinenzartikels entspricht, erstreckt. Die vordere 4a und die hintere Querkante 4b des Absorptionskörpers 4 verlaufen hier bereichsweise gerade. Alternativ können die vordere 4a und/oder die hintere Querkante 4b des Absorptionskörpers auch vollständig gerade, abgewinkelt, gekrümmt, gebogen, wellenförmig oder mit einer anderen dem Fachmann als geeignet erscheinenden Linienführung verlaufen.

Ein vorderer A, mittlerer B und hinterer Bereich C sind beispielhaft auf der Längsmittelachse LMA des Absorptionskörpers 4 zu verorten.

Der vordere Bereich A ist näher an der vorderen Querkante 4a des Absorptionskörpers 4 angeordnet als der mittlere B und der hintere Bereich C. Der hintere Bereich C ist näher an der hinteren Querkante 4b des Absorptionskörpers 4 angeordnet als der mittlere B und vordere Bereich A. Der mittlere Bereich B ist im dargestellten Beispiel in dem Miktionsbereich 9 zu verorten, in welchem im Benutzungszustand Körperflüssigkeiten wie Urin vorrangig auf den Inkontinenzartikel 1a auftreffen.

In diesem Ausführungsbeispiel ist das pH-Regulationsmittel 7 im vorderen vLA, mittleren mLA und hinteren Längsabschnitt hLA des Absorptionskörpers 4 angeordnet, dabei über die gesamte Längserstreckung L des Absorptionskörpers 4 hinweg und dabei auch im Miktionsbereich 9.

Der mittlere mLA, vordere, vLA und hintere Längsabschnitt hLA des Absorptionskörpers 4 entsprechen in ihrer planen Ausdehnung entlang der Längsrichtung 11 und Querrichtung 10 des Absorptionskörpers 4 in diesem Beispiel jeweils einem mittleren, vorderen und hinteren Längsabschnitt des ersten Bereichs 22 der ersten Saugkörperlage 5.

Die Längserstreckung eines mittleren Längsabschnitts mLA des Absorptionskörpers 4 beträgt in diesem Beispiel 62 % der Gesamtlängserstreckung L des Absorptionskörpers 4.

In diesem Ausführungsbeispiel entspricht eine Quermittelachse QMA1 des Inkontinenzartikels 1g nicht einer Quermittelachse QMA2 des Absorptionskörpers 4. Der Absorptionskörper 4 und folglich auch die Quermittelachse QMA2 des Absorptionskörpers 4 sind näher an einer hinteren Querkante 68 des Inkontinenzartikels 1g angeordnet als an einer vorderen Querkante 69 des Inkontinenzartikels 1g. Alternativ kann der Absorptionskörper 4 und folglich auch die Quermittelachse QMA2 des Absorptionskörpers 4 gleich weit zur vorderen 69 und zur hinteren Querkante 68 oder näher an der vorderen 69 als an der hinteren Querkante 69 des Inkontinenzartikels 1g angeordnet sein.

Das pH-Regulationsmittel 7 ist ungleichförmig in die erste Saugkörperlage 5 eingebracht derart, dass im gezeigten Fall die Konzentration und die flächenbezogene Menge an pH-Regulationsmittel 7 in g/m² größer ist im mittleren Längsabschnitt mLA als im vorderen vLA und im hinteren Längsabschnitt hLA des Absorptionskörpers 4.

Bei dem Inkontinenzartikel 1g ist eine Längsrichtung 11 und eine Querrichtung 10 des Inkontinenzartikels 1g, des Chassis 8, des Absorptionskörpers 4 und der ersten Saugkörperlage 5 unterscheidbar, wobei die Querrichtung 10 im angelegten Zustand des Inkontinenzartikels 1g in einem Vorderbereich 82 und einem Rückenbereich 64 des Chassis 8 einer Hüftumfangsrichtung des Benutzers entspricht. Zwischen dem Vorderbereich 82 und dem Rückenbereich 62 ist ein Schrittbereich 84 angeordnet.

An einem jeweiligen Längsrand 85 des Schrittbereichs 84 angrenzend weist das Chassis 8 je einen elastifizierten Abschnitt, mithin einen elastifizierten Beinöffnungsabschnitt 86 auf. Diese elastifizierten Beinöffnungsabschnitte 86 sind im dargestellten Fall gebildet durch zwischen Topsheet 2 und Backsheet 3 verlaufende und in vorgespanntem Zustand an Topsheet 2 und/oder Backsheet 3 fixierte elastische Fäden, die bogenförmig gekrümmt, mithin zumindest mit einer Komponente in Längsrichtung 11 des Inkontinenzartikels 1g orientiert sind.

Der Vorderbereich 82 weist vordere seitliche Längsränder 83 auf, und der Rückenbereich 64 weist hintere seitliche Längsränder 66 auf.

Bei diesem als T-förmige Inkontinenzwindel ausgebildeten Inkontinenzartikel 1g ist im Rückenbereich 64 in Querrichtung 10 beidseitig ein seitlich über den jeweiligen hinteren seitlichen Längsrand 66 hinaus erstrecktes elastisches Windelseitenteil 62,63 vorgesehen, das im Bereich des jeweiligen hinteren seitlichen Längsrandes 66 in einem Überlappungsbereich 87 unlösbar an den Rückenbereich 64 des Chassis 8 angefügt ist. Im Vorderbereich 82 sind hingegen keine Windelseitenteile vorgesehen.

Die elastischen Windelseitenteile 62,63 der als T-förmige Inkontinenzwindel ausgebildeten Inkontinenzartikels 1g haben im Bereich ihres in Querrichtung 10 freien Endes 88 jeweils wenigstens ein Verschlusselement 44,45. Das Verschlusselement 44,45 ist in Form einer vorzugsweise rechteckigen Lasche ausgebildet und herstellerseitig auf sich selbst eingefaltet. In der Gebrauchssituation lässt sich das Verschlusselement 44,45 öffnen, das heißt wieder auffalten, um den Inkontinenzartikel 1g an einen Benutzer anzulegen, wobei die elastischen Windelseitenteile 62,63 in Überlappung mit dem Vorderbereich 82 des Chassis 8 gebracht werden und die Verschlusselemente 44,45 lösbar haftend auf einer vom Benutzer abgewandten Seite des Vorderbereichs 82 des Chassis 8 festgelegt werden. Das elastische Windelseitenteil 62 ist in der Figur 4 in einem ungefalteten Zustand und das elastische Windelseitenteil 63 in einem zweifach auf sich selbst gefalteten Zustand dargestellt.

In einer alternativen Ausführungsvariante kann der Inkontinenzartikel auch als H-förmige Inkontinenzwindel ausgebildet sein, wobei dann zusätzlich im Vorderbereich und bevorzugt beidseitig Windelseitenteile ausgebildet sind, wie das beispielsweise in WO2005102241A1 gezeigt ist. Als weitere Ausführungsvariante ist auch eine Inkontinenzwindel des geschlossenen Typs denkbar, wobei solchen Falls vorzugsweise im Vorderbereich und im Rückenbereich jeweils ein Bauch- bzw. Rückenteil angefügt ist, welche an jeweiligen seitlichen Längsrändern des Bauch- und Rückenteils miteinander gefügt sein derart, dass die Inkontinenzwindel in Hüftumfangsrichtung ringförmig geschlossen ist, wie beispielsweise in WO2013171068A1 gezeigt. Als weitere Ausführungsvariante kann der Inkontinenzartikel als Krankenunterlage oder Inkontinenzvorlage ausgebildet sein.

Weiter kann der Inkontinenzartikel optional einen hier nicht dargestellten in der Längsrichtung orientierten und bevorzugt auf einer Längsmittelachse des Inkontinenzartikels angeordneten Kanal aufweisen, wie mit Bezug zu **Figuren 1a** und **3a**,**b** näher beschrieben, wobei der Kanal zumindest eine Teilmenge des pH-Regulationsmittels aufweist. Solchenfalls ist der Kanal von dem ersten Bereich der ersten Saugkörperlage vollständig umgeben, wobei der erste Bereich die gesamte Menge des SAP der ersten Saugkörperlage aufweist und der Kanal im Wesentlichen frei von SAP ist. Alternativ kann der Inkontinenzartikel auch mehr als einen Kanal, insbesondere zwei Kanäle aufweisen.

### Testmethode pH-Wert-Messung an der Oberfläche eines Inkontinenzartikels:

Zur Durchführung einer pH-Wert-Messung an der Oberfläche eines Inkontinenzartikels, insbesondere auf einer äußeren Oberseite des Topsheets des Inkontinenzartikels, wird eine Urinersatzlösung verwendet. Die Urinersatzlösung ist eine nach ISO 11984-1 (1996) hergestellte 0,9 %ige (w/v) Lösung aus Natriumchlorid (NaCl) in destilliertem Wasser, welche mit Natriumhydroxid (NaOH) auf pH 6.8 eingestellt ist. Eine solche Lösung ist im allgemeinen Fachwissen seit langem bekannt und deren Herstellung gehört zu den Routinemethoden des Fachgebiets.

Der zu testende Inkontinenzartikel wird mit der Topsheet-Seite nach oben flach ausgebreitet, dabei gegebenenfalls vollständig aufgefaltet und gegebenenfalls auf einer Unterlage fixiert. Der Test wird entsprechend der ISO 11984-1 (1996) bei einer Temperatur von 23 °C ± 2 °C und einer relativen Luftfeuchtigkeit von 50% ± 5% durchgeführt. Der zu testende Inkontinenzartikel und die Urinersatzflüssigkeit werden gemäß ISO 11984-1 (1996) entsprechend vorkonditioniert.

Um eine Benutzungssituation relativ realitätsnah abzubilden und die pH-Wert-Entwicklung oder die Pufferwirkung des pH-Regulationsmittels über eine längere Benutzungsdauer mit mehreren Miktionsereignissen zu verfolgen, werden wie nachfolgend beschrieben im Verlauf des Tests drei Miktionsereignisse über einen Zeitraum von fünf Stunden simuliert. Der Inkontinenzartikel wird hierbei insgesamt mit einem Flüssigkeitsvolumen beladen, das etwa 90% einer maximalen Absorptionskapazität des Inkontinenzartikels entspricht.

Die Volumina der ersten und zweiten und dritten simulierten Miktion sind daher so zu wählen, dass ein Gesamtvolumen der ersten und zweiten und dritten simulierten Miktion etwa 90% der maximalen Absorptionskapazität des Inkontinenzartikels entspricht. Dabei entspricht ein Volumen einer ersten simulierten Miktion 45% bis 50% der maximalen Absorptionskapazität des Inkontinenzartikels. Ein jeweiliges Volumen einer zweiten und einer dritten simulierten Miktion ist im Wesentlichen gleich und entspricht jeweils 20% bis 22,5% der maximalen Absorptionskapazität.

Falls die maximale Absorptionskapazität eines Inkontinenzartikels nicht bekannt ist, wird die maximale Absorptionskapazität anhand eines zweiten vergleichbaren Inkontinenzartikels, beispielsweise eines derselben Saugstärke und Größe und Produktart zuzuordnenden und gegebenenfalls derselben Packung zu entnehmenden Inkontinenzartikels wie der zu testende Inkontinenzartikel, vorab gemäß ISO 11984-1 (1996) bestimmt.

Ein Volumen der Urinersatzlösung, welches 50% der maximalen Absorptionskapazität des Inkontinenzartikels entspricht, wird innerhalb von 30 Sekunden zum Zweck der Simulierung einer ersten Miktion auf den trockenen Inkontinenzartikel gegossen und zwar derart, dass die Urinersatzlösung in einem Bereich auf den Inkontinenzartikel trifft, in welchem auch bei einem Miktionsereignis im Benutzungszustand die Miktionsflüssigkeit auf den Inkontinenzartikel treffen würde. Sollte dieser Bereich nicht zweifelsfrei feststehen, soll die Urinersatzlösung mittig auf den Absorptionskörper, insbesondere im Bereich einer Quermittelachse des Absorptionskörpers treffen. Falls der Inkontinenzartikel eine Flüssigkeitsaufnahme- und Verteilerschicht (ADL) aufweist, wird die Urinersatzlösung insbesondere mittig innerhalb eines von der ADL gebildeten Bereiches auf den Inkontinenzartikel gegossen.

Zwei Stunden nach Beginn der ersten simulierten Miktion mit dem ersten Volumen der Urinersatzlösung wird ein zweites Volumen der Urinersatzlösung, welches 20 % der maximalen Absorptionskapazität des Inkontinenzartikels entspricht, wie oben beschrieben auf den Inkontinenzartikel gegossen, um eine zweite Miktion zu simulieren.

Fünf Stunden nach Beginn der Beladung mit dem ersten Volumen der Urinersatzlösung wird ein drittes Volumen der Urinersatzlösung, welches 20 % der maximalen Absorptionskapazität des Inkontinenzartikels entspricht, wie oben beschrieben auf den Inkontinenzartikel gegossen, um eine dritte Miktion zu simulieren.

Nach jedem der drei simulierten Miktionsereignisse wird der pH-Wert an der äußeren Oberseite des Topsheets des Inkontinenzartikels 1, 3, 5, 10, 15 und 20 min nach Ende der jeweiligen simulierten Miktion gemessen. Dazu wird der pH-Wert an jeweils 4 Messpunkten gemessen, welche in Längs- und/oder Querrichtung des flach ausgebreiteten Inkontinenzartikels voneinander beabstandet sind und ein Durchschnittswert der vier gemessenen Werte gebildet. Falls eine ADL vorhanden ist, sollen die Messungen innerhalb des von der ADL gebildeten Bereiches erfolgen.

In jedem Fall sind die Messpunkte innerhalb eines von pH-Regulationsmittel überfangenen Bereiches anzuordnen. Die Messpunkte sollen voneinander beabstandet sein, insbesondere zumindest nicht miteinander überlappen.

Es ist grundsätzlich darauf zu achten, dass die Messpunkte des Inkontinenzartikels nicht zu trocken sind, um verlässliche Messergebnisse zu erhalten. Bevorzugt sollen die Positionen der 4 Messpunkte bei allen Messungen beibehalten werden, jedoch ist auch möglich, die Position eines oder mehrerer Messpunkte zu variieren, beispielsweise falls ein Messpunkt zum Zeitpunkt einer späteren Messung zu trocken sein sollte. Das kann beispielsweise bei Inkontinenzartikeln mit besonders niedriger Rücknässung insbesondere 15 und/oder 20 Minuten nach der jeweiligen simulierten Miktion der Fall sein. Sollten nicht wenigstens zwei geeignete Messpunkte für eine Messung zu einem bestimmten Zeitpunkt zur Verfügung stehen, wird die Messung nur zu den jeweils anderen Zeitpunkten nach Ende der jeweiligen simulierten Miktion ausgewertet. Sollten für eine Messung nur zwei oder drei Messpunkte zur Verfügung stehen ist ein Durchschnittswert der zwei oder drei gemessenen Werte zu bilden. Aufgrund des Routine-Charakters solcher pH-Messungen im Fachgebiet ist es dem Fachmann leicht möglich, eine Beurteilung vorzunehmen, ob ein möglicher Messpunkt zu trocken ist.

Die Messung kann grundsätzlich mit jedem Messgerät vorgenommen werden, das sich nach Einschätzung des Fachmanns für die Oberflächenmessung von pH-Werten eignet.

Beispielsweise eignen sich die Elektroden der Modelle HI14142 oder HI1413 von HANNA Instruments. Das Messgerät wird vor Beginn der Messung entsprechend der Herstellerangaben kalibriert.

## Patentansprüche

1. Inkontinenzartikel für die Aufnahme von Körperausscheidungen, umfassend ein zumindest bereichsweise flüssigkeitsdurchlässiges Topsheet (2), ein im Wesentlichen flüssigkeitsundurchlässiges Backsheet (3) und einen zwischen dem Topsheet (2) und dem Backsheet (3) angeordneten Absorptionskörper (4) wobei der Absorptionskörper (4) eine erste Saugkörperlage (5) aufweist, und wobei ein pH-Regulationsmittel (7) ungleichförmig in die erste Saugkörperlage (5) eingebracht ist, so dass ein mittlerer Bereich des Absorptionskörpers (4) und ein vorderer Bereich des Absorptionskörpers (4) und ein hinterer Bereich des Absorptionskörpers (4) pH-Regulationsmittel (7) enthalten, derart, dass eine flächenbezogene Menge des pH-Regulationsmittels (7) in dem mittleren Bereich des Absorptionskörpers (4) in g/m² größer ist als in dem vorderen und in dem hinteren Bereich des Absorptionskörpers (4).

2. Inkontinenzartikel nach Anspruch 1 **dadurch gekennzeichnet dass** die flächenbezogene Menge des pH-Regulationsmittels (7) in einem mittleren Längsabschnitt des Absorptionskörpers (4) in g/m² größer ist als in einem vorderen und/oder einem hinteren Längsabschnitt des Absorptionskörpers (4).

3. Inkontinenzartikel nach Anspruch 1 oder 2 **dadurch gekennzeichnet dass** 5,00-30,00 g/m², insbesondere 6,00-25,00 g/m², weiter insbesondere 7,00-20,00 g/m², weiter insbesondere 8,00-15,00 g/m² pH-Regulationsmittel (7) im mittleren Längsabschnitt des Absorptionskörpers (4) angeordnet ist und wobei im vorderen und/oder hinteren Längsabschnitt des Absorptionskörpers (4) jeweils 0,00-10,00 g/m², insbesondere 0,10-9,00 g/m², weiter insbesondere 0,20-7,00 g/m², weiter insbesondere 0,30-5,00 g/m² pH-Regulationsmittel (7) angeordnet ist.

4. Inkontinenzartikel nach Anspruch 2 oder 3 **dadurch gekennzeichnet dass** das pH-Regulationsmittel (7) in der ersten Saugkörperlage (5) derart eingebracht ist, dass die Konzentration des pH-Regulationsmittels (7) in Gewichtsprozent in dem mittleren Längsabschnitt des Absorptionskörpers (4) größer ist als in dem vorderen und/oder dem hinteren Längsabschnitt des Absorptionskörpers (4).

5. Inkontinenzartikel nach einem oder mehreren der vorstehenden Ansprüche **dadurch gekennzeichnet dass** die erste Saugkörperlage (5) SAP (20) aufweist.

6. Inkontinenzartikel nach Anspruch 5 **dadurch gekennzeichnet dass** die erste Saugkörperlage (5) einen ersten Bereich (22) aufweist, wobei das SAP (20) in dem ersten Bereich (22) der ersten Saugkörperlage (5) derart eingebracht ist, dass die Konzentration des SAP (20) in Gewichtsprozent in einem mittleren Längsabschnitt des ersten Bereichs (22) der ersten Saugkörperlage (5) gleich groß ist wie in einem vorderen und/oder einem hinteren Längsabschnitt des ersten Bereichs (22) der ersten Saugkörperlage (5).

7. Inkontinenzartikel nach einem oder mehreren der vorstehenden Ansprüche **dadurch gekennzeichnet dass** die erste Saugkörperlage (5) einen in der Längsrichtung orientierten Kanal (16) aufweist, wobei zumindest eine Teilmenge des pH-Regulationsmittels (7) in den Kanal (16) eingebracht ist.

8. Inkontinenzartikel nach Anspruch 7 **dadurch gekennzeichnet, dass** der erste Bereich (22) an den Kanal (16) angrenzt, insbesondere dass der erste Bereich (22) den Kanal (16) vollständig umgibt und wobei zumindest eine Teilmenge des SAP (20), insbesondere die gesamte Menge des SAP (20) der ersten Saugkörperlage (5) in den ersten Bereich (22) eingebracht ist.

9. Inkontinenzartikel nach einem oder mehreren der vorstehenden Ansprüche **dadurch gekennzeichnet dass** der Absorptionskörper (4) eine zweite Saugkörperlage (6) aufweist, wobei die zweite Saugkörperlage (6) unterhalb der ersten Saugkörperlage (5), also zwischen erster Saugkörperlage (5) und Backsheet (3), angeordnet ist.

10. Inkontinenzartikel nach Anspruch 9 **dadurch gekennzeichnet dass** die zweite Saugkörperlage (6) weniger als 20 Gewichtsprozent, 15 Gewichtsprozent, 10 Gewichtsprozent, 5 Gewichtsprozent SAP (20) aufweist, insbesondere frei von SAP (20) ist.

11. Inkontinenzartikel nach einem oder mehreren der Ansprüche 7 bis 10 **dadurch gekennzeichnet dass** der Kanal (16) eine sich durch die erste Saugkörperlage (5) in Dickenrichtung (21) hindurcherstreckende Ausnehmung umfasst oder daraus gebildet ist.

12. Inkontinenzartikel nach einem oder mehreren der Ansprüche 7 bis 11 **dadurch gekennzeichnet dass** der Kanal (16) auf einer Längsmittelachse des Inkontinenzartikels angeordnet ist.

13. Inkontinenzartikel nach einem oder mehreren der vorstehenden Ansprüche **dadurch gekennzeichnet dass** das pH-Regulationsmittel (7) partikelförmig ausgebildet ist.

14. Inkontinenzartikel nach Anspruch 13 **dadurch gekennzeichnet dass** mindestens 50 Gewichtsprozent des pH-Regulationsmittels (7) eine Partikelgröße von 10 bis 2000 µm, insbesondere von 50 bis 1200 µm, weiter insbesondere von 80 bis 800 µm aufweist.

15. Inkontinenzartikel nach einem oder mehreren der Ansprüche 7 bis 14 **dadurch gekennzeichnet dass** die flächenbezogene Menge an pH-Regulationsmittel (7) in dem Kanal (16) 5 bis 100 g/m², insbesondere 10 bis 50 g/m², weiter insbesondere 15 bis 30 g/m² beträgt.

16. Inkontinenzartikel nach einem oder mehreren der vorstehenden Ansprüche **dadurch gekennzeichnet dass** das pH-Regulationsmittel (7) Trinatriumcitrat, Mononatriumcitrat oder Dinatriumcitrat aufweist.

17. Inkontinenzartikel nach Anspruch 16, wobei das pH-Regulationsmittel (7) aus Mononatriumcitrat oder Dinatriumcitrat besteht.

18. Inkontinenzartikel nach einem oder mehreren der Ansprüche 6 bis 17 **dadurch gekennzeichnet dass** die erste Saugkörperlage (5) in dem ersten Bereich (22) zumindest eine Teilmenge des pH-Regulationsmittels (7) aufweist.

19. Inkontinenzartikel nach einem oder mehreren der Ansprüche 7 bis 18 **dadurch gekennzeichnet dass** der Inkontinenzartikel mindestens eine Flüssigkeitsaufnahme- und Verteilerschicht (ADL) (15) aufweist, welche zwischen dem Topsheet (2) und der ersten Saugkörperlage (5) angeordnet ist und wobei die ADL (15) den Kanal (16) bereichsweise, insbesondere vollständig überfängt.

20. Inkontinenzartikel nach einem oder mehreren der vorhergehenden Ansprüche **dadurch gekennzeichnet dass** der Inkontinenzartikel über mindestens zwei, insbesondere mindestens drei Miktionsereignisse hinweg, einen hautfreundlichen pH-Wert aufrechterhält.

21. Inkontinenzartikel nach Anspruch 20 **dadurch gekennzeichnet dass** der pH-Wert auf einer der Haut zugewandten Oberseite des Inkontinenzartikels einen Wert von 4,0-6,5, insbesondere 4,2-6,2, weiter insbesondere 4,3-6,0, weiter insbesondere 4,5-5,8, weiter insbesondere 4,7-5,7 beträgt.

22. Inkontinenzartikel nach einem oder mehreren der Ansprüche 7 bis 21 **dadurch gekennzeichnet dass** der Kanal (16) der ersten Saugkörperlage (5) im Wesentlichen frei von SAP (20) ist.

23. Inkontinenzartikel nach einem oder mehreren der Ansprüche 9 bis 22, **dadurch gekennzeichnet dass** die zweite Saugkörperlage (6) weniger als 20 Gewichtsprozent, insbesondere weniger als 15 Gewichtsprozent, weiter insbesondere weniger als 10 Gewichtsprozent, weiter insbesondere weniger als 5 Gewichtsprozent des pH-Regulationsmittels (7) aufweist, insbesondere frei von pH-Regulationsmittel (7) ist.

## Claims

1. Incontinence article for absorption of bodily excretions, comprising an at least regionally liquid-permeable topsheet (2), a substantially liquid-impermeable backsheet (3), and an absorption body (4) arranged between the topsheet (2) and the backsheet (3), wherein the absorption body (4) comprises a first absorbent ply (5), and wherein a pH regulator (7) is nonuniformly introduced into the first absorbent ply (5), so that a middle region of the absorption body (4) and a front region of the absorption body (4) and a rear region of the absorption body (4) contain pH regulator (7) such that an amount per unit area of the pH regulator (7) is, in g/m², greater in the middle region of the absorption body (4) than in the front region and in the rear region of the absorption body (4).

2. Incontinence article according to Claim 1, **characterized in that** the amount per unit area of the pH regulator (7) is, in g/m², greater in a middle longitudinal section of the absorption body (4) than in a front longitudinal section and/or a rear longitudinal section of the absorption body (4).

3. Incontinence article according to Claim 1 or 2, **characterized in that** 5.00-30.00 g/m², in particular 6.00-25.00 g/m², further in particular 7.00-20.00 g/m², further in particular 8.00-15.00 g/m² of pH regulator (7) is arranged in the middle longitudinal section of the absorption body (4) and wherein there is arranged in the front longitudinal section and/or rear longitudinal section of the absorption body (4), in each case, 0.00-10.00 g/m², in particular 0.10-9.00 g/m², further in particular 0.20-7.00 g/m², further in particular 0.30-5.00 g/m² of pH regulator (7).

4. Incontinence article according to Claim 2 or 3, **characterized in that** the pH regulator (7) is introduced in the first absorbent ply (5) such that the concentration of the pH regulator (7) in percent by weight is greater in the middle longitudinal section of the absorption body (4) than in the front longitudinal section and/or the rear longitudinal section of the absorption body (4).

5. Incontinence article according to one or more of the preceding claims, **characterized in that** the first absorbent ply (5) comprises a SAP (20).

6. Incontinence article according to Claim 5, **characterized in that** the first absorbent ply (5) has a first region (22), wherein the SAP (20) is introduced in the first region (22) of the first absorbent ply (5) such that the concentration of the SAP (20) in percent by weight in a middle longitudinal section of the first region (22) of the first absorbent ply (5) is the same as in a front longitudinal section and/or a rear longitudinal section of the first region (22) of the first absorbent ply (5).

7. Incontinence article according to one or more of the preceding claims, **characterized in that** the first absorbent ply (5) comprises a channel (16) oriented in the longitudinal direction, wherein at least a partial amount of the pH regulator (7) is introduced into the channel (16).

8. Incontinence article according to Claim 7, **characterized in that** the first region (22) adjoins the channel (16) and, in particular, **in that** the first region (22) completely surrounds the channel (16) and wherein at least a partial amount of the SAP (20), in particular the total amount of the SAP (20), of the first absorbent ply (5) is introduced into the first region (22).

9. Incontinence article according to one or more of the preceding claims, **characterized in that** the absorption body (4) comprises a second absorbent ply (6), wherein the second absorbent ply (6) is arranged below the first absorbent ply (5), i.e., between first absorbent ply (5) and backsheet (3).

10. Incontinence article according to Claim 9, **characterized in that** the second absorbent ply (6) comprises less than 20 percent by weight, 15 percent by weight, 10 percent by weight, 5 percent by weight of SAP (20), and in particular is free of SAP (20).

11. Incontinence article according to one or more of Claims 7 to 10, **characterized in that** the channel (16) comprises or is formed from a cutout which extends through the first absorbent ply (5) in the thickness direction (21).

12. Incontinence article according to one or more of Claims 7 to 11, **characterized in that** the channel (16) is arranged on a longitudinal central axis of the incontinence article.

13. Incontinence article according to one or more of the preceding claims, **characterized in that** the pH regulator (7) is particulate.

14. Incontinence article according to Claim 13, **characterized in that** at least 50 percent by weight of the pH regulator (7) has a particle size of 10 to 2000 µm, in particular of 50 to 1200 µm, further in particular of 80 to 800 µm.

15. Incontinence article according to one or more of Claims 7 to 14, **characterized in that** the amount per unit area of pH regulator (7) in the channel (16) is 5 to 100 g/m², in particular 10 to 50 g/m², further in particular 15 to 30 g/m².

16. Incontinence article according to one or more of the preceding claims, **characterized in that** the pH regulator (7) comprises trisodium citrate, monosodium citrate or disodium citrate.

17. Incontinence article according to Claim 16, wherein the pH regulator (7) consists of monosodium citrate or disodium citrate.

18. Incontinence article according to one or more of Claims 6 to 17, **characterized in that** the first absorbent ply (5) in the first region (22) comprises at least a partial amount of the pH regulator (7).

19. Incontinence article according to one or more of Claims 7 to 18, **characterized in that** the incontinence article comprises at least one acquisition distribution layer (ADL) (15) which is arranged between the topsheet (2) and the first absorbent ply (5) and wherein the ADL (15) covers the channel (16) regionally, in particular completely.

20. Incontinence article according to one or more of the preceding claims, **characterized in that** the incontinence article maintains a skin-friendly pH over at least two, in particular at least three, micturition events.

21. Incontinence article according to Claim 20, **characterized in that** the pH on a topside of the incontinence article that faces the skin is a value of 4.0-6.5, in particular 4.2-6.2, further in particular 4.3-6.0, further in particular 4.5-5.8, further in particular 4.7-5.7.

22. Incontinence article according to one or more of Claims 7 to 21, **characterized in that** the channel (16) of the first absorbent ply (5) is substantially free of SAP (20).

23. Incontinence article according to one or more of Claims 9 to 22, **characterized in that** the second absorbent ply (6) comprises less than 20 percent by weight, in particular less than 15 percent by weight, further in particular less than 10 percent by weight, further in particular less than 5 percent by weight of the pH regulator (7) and in particular is free of pH regulator (7).

## Revendications

1. Article d'incontinence destiné à absorber des excrétions corporelles, comprenant une feuille supérieure (2) au moins partiellement perméable aux liquides, une feuille arrière (3) sensiblement imperméable aux liquides et un corps d'absorption (4) agencé entre la feuille supérieure (2) et la feuille arrière (3), le corps d'absorption (4) présentant une première couche de corps absorbant (5) et un agent de régulation du pH (7) étant incorporé de manière non uniforme dans la première couche de corps absorbant (5) de telle sorte qu'une zone centrale du corps d'absorption (4) et une zone avant du corps d'absorption (4) et une zone arrière du corps d'absorption (4) contiennent de l'agent de régulation du pH (7) de manière telle qu'une quantité, rapportée à la surface, de l'agent de régulation du pH (7) dans la zone centrale du corps d'absorption (4), en g/m², est supérieure à celle dans la zone avant et dans la zone arrière du corps d'absorption (4).

2. Article d'incontinence selon la revendication 1, **caractérisé en ce que** la quantité, rapportée à la surface, de l'agent de régulation du pH (7) dans une section longitudinale centrale du corps d'absorption (4), en g/m², est supérieure à celle dans une section longitudinale avant et/ou arrière du corps d'absorption (4).

3. Article d'incontinence selon la revendication 1 ou 2, **caractérisé en ce que** 5,00-30,00 g/m², en particulier 6,00-25,00 g/m², plus particulièrement 7,00-20,00 g/m², plus particulièrement 8,00-15,00 g/m² d'agent de régulation du pH (7) sont agencés dans la section longitudinale centrale du corps d'absorption (4) et à chaque fois 0,00-10,00 g/m², en particulier 0,10-9,00 g/m², plus particulièrement 0,20-7,00 g/m², plus particulièrement 0,30-5,00 g/m² d'agent de régulation du pH (7) sont agencés dans la section longitudinale avant et/ou arrière du corps d'absorption (4).

4. Article d'incontinence selon la revendication 2 ou 3, **caractérisé en ce que** l'agent de régulation du pH (7) est incorporé dans la première couche de corps absorbant (5) de manière telle que la concentration en agent de régulation du pH (7), en pourcentage en poids, dans la section longitudinale centrale du corps d'absorption (4) est supérieure à celle dans la section longitudinale avant et/ou arrière du corps d'absorption (4).

5. Article d'incontinence selon l'une ou plusieurs des revendications précédentes, **caractérisé en ce que** la première couche de corps absorbant (5) présente un SAP (polymère superabsorbant) (20).

6. Article d'incontinence selon la revendication 5, **caractérisé en ce que** la première couche de corps absorbant (5) présente une première zone (22), le SAP (20) étant incorporé dans la première zone (22) de la première couche de corps absorbant (5) de manière telle que la concentration en SAP (20), en pourcentage en poids, dans une section longitudinale centrale de la première zone (22) de la première couche de corps absorbant (5) est identique à celle dans une section longitudinale avant et/ou arrière de la première zone (22) de la première couche de corps absorbant (5).

7. Article d'incontinence selon l'une ou plusieurs des revendications précédentes, **caractérisé en ce que** la première couche de corps absorbant (5) présente un canal (16) orienté dans la direction longitudinale, au moins une quantité partielle de l'agent de régulation du pH (7) étant incorporée dans le canal (16).

8. Article d'incontinence selon la revendication 7, **caractérisé en ce que** la première zone (22) est adjacente au canal (16), en particulier **en ce que** la première zone (22) entoure complètement le canal (16) et au moins une quantité partielle du SAP (20), en particulier la quantité totale du SAP (20), de la première couche de corps absorbant (5) étant incorporée dans la première zone (22).

9. Article d'incontinence selon l'une ou plusieurs des revendications précédentes, **caractérisé en ce que** le corps d'absorption (4) présente une deuxième couche de corps absorbant (6), la deuxième couche de corps absorbant (6) étant agencée sous la première couche de corps absorbant (5), c'est-à-dire entre la première couche de corps absorbant (5) et la feuille arrière (3).

10. Article d'incontinence selon la revendication 9, **caractérisé en ce que** la deuxième couche de corps absorbant (6) présente moins de 20% en poids, moins de 15% en poids, moins de 10% en poids, moins de 5% en poids de SAP (20) et est en particulier exempte de SAP (20).

11. Article d'incontinence selon l'une ou plusieurs des revendications 7 à 10, **caractérisé en ce que** le canal (16) comprend ou est formé par un évidement s'étendant à travers la première couche de corps absorbant (5) dans la direction de l'épaisseur (21).

12. Article d'incontinence selon l'une ou plusieurs des revendications 7 à 11, **caractérisé en ce que** le canal (16) est agencé sur un axe central longitudinal de l'article d'incontinence.

13. Article d'incontinence selon l'une ou plusieurs des revendications précédentes, **caractérisé en ce que** l'agent de régulation du pH (7) est réalisé sous forme de particules.

14. Article d'incontinence selon la revendication 13, **caractérisé en ce qu'**au moins 50% en poids de l'agent de régulation du pH (7) présentent une grosseur de particule de 10 à 2000 µm, en particulier de 50 à 1200 µm, plus particulièrement de 80 à 800 µm.

15. Article d'incontinence selon l'une ou plusieurs des revendications 7 à 14, **caractérisé en ce que** la quantité, rapportée à la surface, d'agent de régulation du pH (7) dans le canal (16) est de 5 à 100 g/m², en particulier de 10 à 50 g/m², plus particulièrement de 15 à 30 g/m².

16. Article d'incontinence selon l'une ou plusieurs des revendications précédentes, **caractérisé en ce que** l'agent de régulation du pH (7) présente du citrate trisodique, du citrate monosodique ou du citrate disodique.

17. Article d'incontinence selon la revendication 16, l'agent de régulation du pH (7) étant constitué par du citrate monosodique ou du citrate disodique.

18. Article d'incontinence selon l'une ou plusieurs des revendications 6 à 17, **caractérisé en ce que** la première couche de corps absorbant (5) présente, dans la première zone (22), au moins une quantité partielle de l'agent de régulation du pH (7).

19. Article d'incontinence selon l'une ou plusieurs des revendications 7 à 18, **caractérisé en ce que** l'article d'incontinence présente au moins une couche d'absorption et de répartition de liquide (ADL) (15), qui est agencée entre la feuille supérieure (2) et la première couche de corps absorbant (5), et l'ADL (15) recouvrant partiellement, en particulier complètement, le canal (16).

20. Article d'incontinence selon l'une ou plusieurs des revendications précédentes, **caractérisé en ce que** l'article d'incontinence conserve une valeur de pH respectueuse de la peau sur au moins deux, en particulier sur au moins trois événements de miction.

21. Article d'incontinence selon la revendication 20, **caractérisé en ce que** la valeur du pH sur une face supérieure orientée vers la peau de l'article d'incontinence présente une valeur de 4,0-6,5, en particulier de 4,2-6,2, plus particulièrement de 4,3-6,0, plus particulièrement de 4,5-5,8, plus particulièrement de 4,7-5,7.

22. Article d'incontinence selon l'une ou plusieurs des revendications 7 à 21, **caractérisé en ce que** le canal (16) de la première couche de corps absorbant (5) est sensiblement exempt de SAP (20).

23. Article d'incontinence selon l'une ou plusieurs des revendications 9 à 22, **caractérisé en ce que** la deuxième couche de corps absorbant (6) présente moins de 20% en poids, en particulier moins de 15% en poids, plus particulièrement moins de 10% en poids, plus particulièrement moins de 5% en poids, de l'agent de régulation du pH (7) et est en particulier exempte d'agent de régulation du pH (7).
